(19) Europäisches Patentamt
European Patent Office
Office européen des brevets

(11) EP 2 168 983 A1

(12) DEMANDE DE BREVET EUROPEEN

(43) Date de publication:
**31.03.2010 Bulletin 2010/13**

(51) Int Cl.:
***C07K 14/665*** *(2006.01)* ***A61K 38/04*** *(2006.01)*

(21) Numéro de dépôt: **08290917.7**

(22) Date de dépôt: **30.09.2008**

(84) Etats contractants désignés:
**AT BE BG CH CY CZ DE DK EE ES FI FR GB GR HR HU IE IS IT LI LT LU LV MC MT NL NO PL PT RO SE SI SK TR**
Etats d'extension désignés:
**AL BA MK RS**

(71) Demandeurs:
- **IPSEN PHARMA**
  **92100 Boulogne-Billancourt (FR)**
- **COMMISSARIAT A L'ENERGIE ATOMIQUE**
  **75015 Paris (FR)**
- **CENTRE NATIONAL DE LA RECHERCHE SCIENTIFIQUE (CNRS)**
  **75794 Paris Cedex 16 (FR)**

(72) Inventeurs:
- **Paternostre, Marie-Thèrèse**
  **91370 Verrières le Buisson (FR)**
- **Cintrat, Jean-Christophe**
  **91430 Igny (FR)**
- **Valery, Céline**
  **08013 Barcelona (ES)**
- **Roux, Stéphane**
  **06100 Nice (FR)**
- **Rousseau, Bernard**
  **92300 Levallois-Perret (FR)**
- **IJsselstijn, Maarten**
  **9731 KL Groningen (NL)**
- **Cherif-Cheikh, Roland**
  **08860 Castelldefels (Barcelona) (ES)**
- **Artzner, Franck**
  **35510 Cesson Sévigné (FR)**

(74) Mandataire: **Bourgouin, André**
**IPSEN - S.C.R.A.S.**
**65 Quai Georges Gorse**
**Direction de la Propriété Intellectuelle**
**92650 Boulogne Billancourt Cédex (FR)**

(54) **Nouveaux composés octapeptidiques et leur utilisation thérapeutique**

(57) La présente invention concerne de nouveaux composés octapeptidiques de formule générale (I).

$$H\text{-}2\text{-}Nal^{1}\text{-}cyclo(Cys^{2}\text{-}Tyr^{3}\text{-}AA^{4}\text{-}Lys^{5}\text{-}Val^{6}\text{-}Cys^{7})\text{-}Thr^{8}\text{-}NH_{2} \qquad (I)$$

Ces produits ayant une bonne affinité pour certains sous-types de récepteurs de la somatostatine, ils sont particulièrement intéressants pour traiter les états pathologiques ou les maladies dans lesquels un (ou plusieurs) des récepteurs de la somatostatine est (sont) impliqué(s). Ces produits ayant également des propriétés d'assemblage particulières, ils sont particulièrement intéressants pour la formulation de médicaments. L'invention concerne donc également des compositions pharmaceutiques contenant lesdits produits et leur utilisation pour la préparation d'un médicament.

EP 2 168 983 A1

## Description

La présente invention concerne de nouveaux composés octapeptidiques. Ces produits ayant une bonne affinité pour certains sous-types de récepteurs de la somatostatine, ils sont particulièrement intéressants pour traiter les états pathologiques ou les maladies dans lesquels un (ou plusieurs) des récepteurs de la somatostatine est (sont) impliqué (s). L'invention concerne également des compositions pharmaceutiques contenant lesdits produits et leur utilisation pour la préparation d'un médicament.

La somatostatine (SST) est un tétradécapeptide cyclique qui a été isolé pour la première fois de l'hypothalamus en tant que substance inhibitrice de l'hormone de croissance (Brazeau P. et al., Science 1973, 179, 77-79). Elle intervient également en tant que neurotransmetteur dans le cerveau (Reisine T. et al., Neuroscience 1995, 67, 777-790 ; Reisine et al., Endocrinology 1995, 16, 427-442). L'hétérogénéité des fonctions biologiques de la somatostatine et les relations structure-activité de ses analogues peptidiques, ont amené la découverte de 5 sous-types de récepteurs liés à la membrane (Yamada et al., Proc. Natl. Acad. Sci. U.S.A, 89, 251-255, 1992 ; Raynor, K. et al, Mol. Pharmacol., 44, 385-392, 1993). Le clonage moléculaire a permis de montrer que la bioactivité de la somatostatine dépend directement de ces cinq sous-types de récepteurs.

Les rôles fonctionnels de ces récepteurs sont actuellement activement étudiés. L'activation préférentielle des sous-types 2 et 5 a été associée à la suppression, dans les adénomes sécrétant ces hormones, de l'hormone de croissance GH (acromégalie), de l'hormone TSH et de la prolactine ; mais le rôle précis de chaque sous-type reste à déterminer.

Parmi les désordres pathologiques associés à la somatostatine (Moreau J.P. et al., Life Sciences, 1987, 40, 419 ; Harris A.G. et al., The European Journal of Medicine, 1993, 2, 97-105), on peut citer par exemple : l'acromégalie, les adénomes hypophysaires, la maladie de Cushing, les gonadotrophinomes et les prolactinomes, les effets secondaires cataboliques des glucocorticoïdes, le diabète, la rétinopathie diabétique, la néphropathie diabétique, l'hyperthyroïdie, le gigantisme, les tumeurs gastroentéropancréatiques endocriniennes dont le syndrôme carcinoïde, le VIPome, l'insulinome, la nésidioblastose, l'hyperinsulinémie, le glucagonome, le gastrinome et le syndrôme de Zollinger-Ellison, le GRFome ainsi que le saignement aigu des varices oesophagiennes, le reflux gastrooesophagien, le reflux gastroduodénal, la pancréatite, les fistules entérocutanées et pancréatiques mais aussi les diarrhées, les diarrhées réfractaires du syndrôme d'immunodépression acquise, la diarrhée chronique sécrétoire, la diarrhée associée avec le syndrôme de l'intestin irrité, les troubles liés au peptide libérateur de gastrine, les pathologies secondaires aux greffes intestinales, l'hypertension portale ainsi que les hémorragies des varices chez des malades avec cirrhose, l'hémorragie gastro-intestinale, l'hémorragie de l'ulcère gastroduodénal, la maladie de Crohn, les scléroses systémiques, le dumping syndrôme, le syndrôme du petit intestin, l'hypotension, la sclérodermie et le carcinome thyroïdien médullaire, les maladies liées à l'hyperprolifération cellulaire comme les cancers et plus particulièrement le cancer du sein, le cancer de la prostate, le cancer thyroïdien ainsi que le cancer pancréatique et le cancer colorectal, les fibroses et plus particulièrement la fibrose du rein, la fibrose du foie, la fibrose du poumon, la fibrose de la peau, également la fibrose du système nerveux central ainsi que celle du nez et la fibrose induite par la chimiothérapie, et d'autres domaines thérapeutiques comme, par exemple, les céphalées y compris les céphalées associées aux tumeurs hypophysaires, les douleurs, les accès de panique, la chimiothérapie, la cicatrisation des plaies, l'insuffisance rénale résultant d'un retard de croissance, l'obésité et retard de croissance lié à l'obésité, le retard de croissance utérin, la dysplasie du squelette, le syndrôme de Noonan, le syndrôme d'apnée du sommeil, la maladie de Graves, la maladie polykystique des ovaires, les pseudokystes pancréatiques et ascites, la leucémie, le méningiome, la cachexie cancéreuse, l'inhibition des H pylori, le psoriasis ainsi que la maladie d'Alzheimer. On peut également citer l'ostéoporose.

De nos jours, une attention croissante est portée aux peptides possédant une affinité sur les récepteurs de la somatostatine. Ainsi, le Lanréotide a été très étudié pour le traitement des maladies liées à l'hormone de croissance (Cendros JM, Peraire C, Trocôniz IF, Obach R. Pharmacokinetics and population pharmacodynamic analysis of lanreotide Autogel. Metabolism. 2005 Oct, 54(10), 1276-81.)

Le besoin de trouver des alternatives aux solutions existantes constitue donc un enjeu majeur. La présente invention s'inscrit dans ce cadre.

La déposante propose donc de nouveaux composés octapeptidiques possédant une bonne affinité sur les récepteurs de la somatostatine et/ou des propriétés physico-chimiques permettant d'envisager des solutions diverses de formulation de médicaments.

Les composés selon l'invention présentent de nombreux avantages, en particulier l'une au moins des caractéristiques suivantes :

- leur affinité sur les récepteurs de la somatostatine,
- leur rhéologie permettant des gammes de viscosités associées liées au confort d'injection,

- leur aptitude à être utilisés comme support de formulation,

- leur faculté d'auto-assemblage sous forme de nanotubes de diamètres variables et monodisperses,

- leur faculté d'autoassemblage sous forme de fibres,

- leurs divers degrés de solubilité dans l'eau.

La présente invention a donc pour objet un composé octapeptidique de formule générale (I)

$$\text{H-2-Nal}^{1}\text{-cyclo(Cys}^{2}\text{-Tyr}^{3}\text{-AA}^{4}\text{-Lys}^{5}\text{-Val}^{6}\text{-Cys}^{7}\text{)-Thr}^{8}\text{-NH}_{2} \qquad \text{(I)}$$

dans laquelle $AA^4$ représente un acide aminé de formule

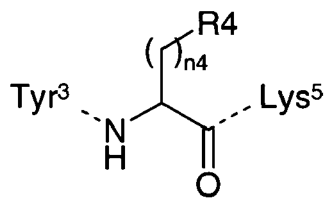

dans laquelle n4 représente un entier de 0 à 3 et R4 représente un atome d'hydrogène, un radical alkyle, aryle ou hétéroaryle, les radicaux aryle et hétéroaryle étant éventuellement substitués par un ou plusieurs radicaux identiques ou différents choisis parmi halo, nitro, hydroxy, aryle, OR41 ;
R41 représente un radical

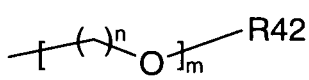

dans lequel R42 représente un radical alkyle ou un atome d'hydrogène, n est un entier de 2 à 4, et m est un entier de 1 à 4 ;
étant entendu que tous les acides aminés peuvent être de configuration D ou L,
à l'exclusion des composés H-D-2-$Nal^1$-cyclo($Cys^2$-$Tyr^3$-β-(3-pyridyl)-D-$Ala^4$-$Lys^5$-$Val^6$-$Cys^7$)-$Thr^8$-$NH_2$, H-D-2-$Nal^1$-cyclo($Cys^2$-$Tyr^3$- D-$Phe^4$- $Lys^5$-$Val^6$-$Cys^7$)-$Thr^8$-$NH_2$, H-D-2-$Nal^1$-cyclo($Cys^2$-$Tyr^3$-D-$Trp^4$- $Lys^5$-$Val^6$-$Cys^7$)-$Thr^8$-$NH_2$ et de leurs sels,
ou un sel pharmaceutiquement acceptable de ce composé.

Au sens de la présente invention, on entend par alkyle, lorsqu'il n'est pas donné plus de précision, un radical alkyle linéaire ou ramifié comptant de 1 à 6 atomes de carbone, tels que méthyle, éthyle, propyle, isopropyle, butyle, isobutyle, tert-butyle, pentyle, ou hexyle, et de préférence de 1 à 4 atomes de carbone.

Par aryle, on entend un système carbocyclique insaturé comprenant au moins un cycle aromatique, et de préférence un radical choisi parmi phényle, naphtyle, anthryle (ou anthracényle) et fluorényle.

Par hétéroaryle au sens de la présente invention, on entend un cycle insaturé aromatique comprenant un ou plusieurs hétéroatomes identiques ou différents choisis parmi N, O et S, tels que les radicaux pyridinyle, pyrimidinyle, furyle, thiènyle, benzothiènyle, oxazolyle, benzoxazolyle, isoxazolyle, thiazolyle, pyrolyle, pyrazolyle, imidazolyle, triazolyle, tétrazolyle et tout particulièrement thiènyle, benzothiènyle et imidazolyle.

Selon la présente invention, l'expression sel pharmaceutiquement acceptable définit des sels d'addition d'acides inorganiques tels que chlorhydrate, bromhydrate, iodhydrate, sulfate, phosphate, diphosphate et nitrate ou d'acides organiques tels que acétate, maléate, fumarate, tartrate, succinate, citrate, lactate, méthanesulfonate, p-toluènesulfonate, pamoate et stéarate. Pour d'autres exemples de sels pharmaceutiquement acceptables, on peut se référer à "Salt selection for basic drugs", Int. J. Pharm. (1986), 33, 201-217.

Selon la présente invention, les acides aminés représentent les acides aminés connus de l'homme de l'art de configuration D ou L, représentés ici selon leur nomenclature habituelle et les analogues synthétiques modifiés sur les chaînes latérales desdits acides aminés parmi lesquels :

- β-(3-benzothiènyl)-Ala, β-(2-thiènyl)-Ala, β-(1-naphthyl)-Ala, β2-naphthyl)-Ala ou 2-Nal, β-(9-anthryl)-Ala, β-(2-fluorenyl)-Ala représentent une alanine substituée en β respectivement par un radical benzothiènyle sur sa position 3, thiènyle sur sa position 2, naphthyle sur sa position 1, naphthyle sur sa position 2, anthryle sur sa position 9, et fluorényle sur sa position 2 ;

- Ph-Gly ou Phg représente une glycine substituée par un radical phényle ;
- Homo-Phe ou Homophe représente une phénylalanine dont la chaîne latérale est allongée d'un chaînon méthylène ; et,
- *p*-Br-Phe, *p*-F-Phe, *p*-Nitro-Phe, *p*-Ph-Phe; *p*-O-2-(2-méthoxyéthoxy)éthoxy-Phe, *m*-Br-Phe, *m*-F-Phe, *o*-Br-Phe, *o*-F-Phe, 3,5-diF-Phe représentent une phénylalanine dont le noyau phényle est substitué respectivement en para par un atome de brome, de fluor, un radical nitro, phényle et O-2-(2-méthoxyéthoxy)éthoxy, en meta par un atome de brome et de fluor, en ortho par un atome de brome et de fluor et en positions 3 et 5 par deux atomes de fluor.

De manière préférentielle, l'invention a plus particulièrement pour objet un composé tel que défini ci-dessus dans lequel n4 représente un entier de 0 à 2 et R4 représente un radical alkyle, aryle ou hétéroaryle, les radicaux aryle et hétéroaryle étant éventuellement substitués par un ou plusieurs radicaux identiques ou différents choisis parmi halo, nitro,
hydroxy, aryle, OR41 ; et R41 représente un radical

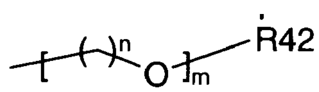

dans lequel
R42 représente un radical alkyle, n et m représentent 2.

L'invention a préférentiellement pour objet un composé tel que défini ci-dessus dans lequel le terme aryle représente un radical choisi parmi phényle, naphtyle, anthryle et fluorényle.

L'invention a préférentiellement également pour objet un composé tel que défini ci-dessus dans lequel le terme hétéroaryle représente un radical choisi parmi pyridinyle, pyrimidinyle, furyle, thiènyle, benzothiènyle, oxazolyle, benzoxazolyle, isoxazolyle, thiazolyle, pyrolyle, pyrazolyle, imidazolyle, triazolyle, tétrazolyle et encore plus préférentiellement, choisi parmi thiènyle, benzothiènyle et imidazolyle.

De même, l'invention a préférentiellement également pour objet un composé tel que défini ci-dessus dans lequel le terme alkyle représente un radical choisi parmi méthyle, éthyle, propyle, isopropyle, butyle, isobutyle, tert-butyle.

De manière plus préférentielle, l'invention concerne un composé octapeptidique de formule générale (I) dans lequel AA$^4$ représente un acide aminé choisi parmi Trp, Ala, β-(3-benzothiényl)-Ala, β-(2-thiényl)-Ala, β-(9-anthryl)-Ala, β-(2-fluorenyl)-Ala, His, Val, 1-Nal, 2-Nal, phényl-Gly, Homo-Phe, *p*-Br-Phe, *p*-F-Phe, *m*-F-Phe, *o*-F-Phe, *m*-Br-Phe, *o*-Br-Phe, *p*-NO$_2$-Phe, 3,5-difuoro-Phe, 4-phényl-Phe, Tyr, *p*-(2-(2-méthoxyéthoxy)éthoxy)-Phe.

De manière encore plus préférentielle, l'invention concerne un composé octapeptidique de formule générale (I) dans laquelle l'acide aminé AA$^4$ est de configuration D et plus préférentiellement encore les acides aminés 2-Nal$^1$ et AA$^4$ sont de configuration D, les autres acides aminés étant de configuration L.

De manière très préférentielle également, l'invention concerne un composé octapeptidique de formule générale (I) dans laquelle l'acide aminé AA$^4$ est de configuration L et plus préférentiellement encore les acides aminés 2-Nal$^1$ et AA$^4$ sont de configuration L, les autres acides aminés étant de configuration D.

Préférentiellement, le composé selon l'invention est choisi parmi :

H-D-2-Nal$^1$-cyclo(Cys$^2$-Tyr$^3$-β-(3-benzothienyl)-D-Ala$^4$-Lys$^5$-Val$^6$-Cys$^7$)-Thr$^8$-NH$_2$
H-D-2-Nal$^1$-cyclo(Cys$^2$-Tyr$^3$-β-(2-thienyl)-D-Ala$^4$-Lys$^5$-Val$^6$-Cys$^7$)-Thr$^8$-NH$_2$
H-D-2-Nal$^1$-cyclo(Cys$^2$-Tyr$^3$-D-His$^4$-Lys$^5$-Val$^6$-Cys$^7$)-Thr$^8$-NH$_2$
H-D-2-Nal$^1$-cyclo(Cys$^2$-Tyr$^3$-D-Ala$^4$-Lys$^5$-Val$^6$-Cys$^7$)-Thr$^8$-NH$_2$
H-D-2-Nal$^1$-cyclo(Cys$^2$-Tyr$^3$-D-Ala$^4$-Lys$^5$-Val$^6$-Cys$^7$)-Thr$^8$-NH$_2$
H-D-2-Na$^1$-cyclo(Cys$^2$-Tyr$^3$-β-(1-naphthyl)-D-Ala$^4$-Lys$^5$-Val$^6$-Cys$^7$)-Thr$^8$-NH2
H-D-2-Na$^1$-cyclo(Cys$^2$-Tyr$^3$-β-(2-naphthyl)-D-Ala$^4$-Lys$^5$-Val$^6$-Cys$^7$)-Thr$^8$-NH$_2$
H-D-2-Na$^1$-cyclo(Cys$^2$-Tyr$^3$-β-(9-naphthyl)-D-Ala$^4$-Lys$^5$-Val$^6$-Cys$^7$)-Thr$^8$-NH$_2$
H-D-2-Na$^1$-cyclo(Cys$^2$-Tyr$^3$-β-(2-naphthyl)-D-Ala$^4$-Lys$^5$-Val$^6$-Cys$^7$)-Thr$^8$-NH$_2$
H-D-2-Nal$^1$-cyclo(Cys$^2$-Tyr$^3$-Ph-D-Gly$^4$-Lys$^S$-Val$^s$-Cys$^7$)-Thr$^8$-NH$_2$
H-D-2-Nal$^1$-cyclo(Cys$^2$-Tyr$^3$-homo-D-Phe$^4$-Lys$^5$-Val$^6$-Cys$^7$)-Thr$^8$-NH$_2$

H-D-2-Nal$^{1}$-cyclo(Cys$^{2}$-Tyr$^{3}$-p-Br-D-Phe$^{4}$-Lys$^{5}$-Val$^{6}$-Cys$^{7}$)-Thr$^{8}$-NH$_{2}$
H-D-2-Nal$^{1}$-cyclo(Cys$^{2}$-Tyr$^{3}$-p-F-D-Phe$^{4}$-Lys$^{5}$-Val$^{6}$-Cys$^{7}$)-Thr$^{8}$-NH$_{2}$
H-D-2-Nal$^{1}$-cyclo(Cys$^{2}$-Tyr$^{3}$-D-Tyr$^{4}$-Lys$^{5}$-Val$^{6}$-Cys$^{7}$)-Thr$^{8}$-NH$_{2}$
H-D-2-Nal$^{1}$-cyclo(Cys$^{2}$-Tyr$^{3}$-*m*-F-D-Phe$^{4}$-Lys$^{5}$-Val$^{6}$-Cys$^{7}$)-Thr$^{8}$-NH$_{2}$
H-D-2-Nal$^{1}$-cyclo(Cys$^{2}$-Tyr$^{3}$-*o*-F-D-Phe$^{4}$-Lys$^{5}$-Val$^{6}$-Cys$^{7}$)-Thr$^{8}$-NH$_{2}$
H-D-2-Na$^{1}$-cyclo(Cys$^{2}$-Tyr$^{3}$-*3,5*-diF-D-Phe$^{4}$-Lys$^{5}$-Val$^{6}$-Cys$^{7}$)-Thr$^{8}$-NH$_{2}$
H-D-2-Nal$^{1}$-cyclo(Cys$^{2}$-Tyr$^{3}$-*m*-Br-D-Phe$^{4}$-Lys$^{5}$-Val$^{6}$-Cys$^{7}$)-Thr$^{8}$-NH$_{2}$
H-D-2-Nal$^{1}$-cyclo(Cys$^{2}$-Tyr$^{3}$-*o*-Br-D-Phe$^{4}$-Lys$^{5}$-Val$^{6}$-Cys$^{7}$)-Thr$^{8}$-NH$_{2}$
H-D-2-Nal$^{1}$-cyclo(Cys$^{2}$-Tyr$^{3}$-*p*-Nitro-D-Phe$^{4}$-Lys$^{5}$-Val$^{6}$-Cys$^{7}$)-Thr$^{8}$-NH$_{2}$
H-D-2-Nal$^{1}$-cyclo(Cys$^{2}$-Tyr$^{3}$-*p*-Ph-D-Phe$^{4}$-Lys$^{5}$-Val$^{6}$-Cys$^{7}$)-Thr$^{8}$-NH$_{2}$
H-D-2-Nal$^{1}$-cyclo(Cys$^{2}$-Tyr$^{3}$-*p*-(2-(2-méthoxyéthoxy)éthoxy)-D-Phe$^{4}$-Lys$^{5}$-Val$^{6}$-Cys$^{7}$)-Thr$^{8}$-NH$_{2}$
H-2-Nal$^{1}$-cyclo(D-Cys$^{2}$-D-Tyr$^{3}$-Trp$^{4}$-D-Lys$^{5}$-D-Val$^{6}$-D-Cys$^{7}$)-D-Thr$^{8}$-NH$_{2}$,
ou un sel pharmaceutiquement acceptable de ce composé.

Plus préférentiellement, le composé selon l'invention est choisi parmi :

H-D-2-Nal$^{1}$-cyclo(Cys$^{2}$-Tyr$^{3}$-β-(3-benzothienyl)-D-Ala$^{4}$-Lys$^{5}$-Val$^{6}$-Cys$^{7}$)-Thr$^{8}$-NH$_{2}$
H-D-2-Nal$^{1}$-cyclo(Cys$^{2}$-Tyr$^{3}$-β-(2-thienyl)-D-Ala$^{4}$-Lys$^{5}$-Val$^{6}$-Cys$^{7}$)-Thr$^{8}$-NH$_{2}$
H-D-2-Nal$^{1}$-cyclo(Cys$^{2}$-Tyr$^{3}$-D-His$^{4}$-Lys$^{5}$ -Val$^{6}$-Cys$^{7}$)-Thr$^{8}$-NH$_{2}$
H-D-2-Nal$^{1}$-cyclo(Cys$^{2}$-Tyr$^{3}$-β-(1-naphthyl)-D-Ala$^{4}$-Lys$^{5}$-Val$^{6}$-Cys$^{7}$)-Thr$^{8}$-NH$_{2}$
H-D-2-Nal$^{1}$-cyclo(Cys$^{2}$-Tyr$^{3}$-β-(2-fluorenyl)-D-Ala$^{4}$-Lys$^{5}$-Val$^{6}$-Cys$^{7}$)-Thr$^{8}$-NH$_{2}$
H-D-2-Nal$^{1}$-cyclo(Cys$^{2}$-Tyr$^{3}$-*p*-Br-D-Phe$^{4}$-Lys$^{5}$-Val$^{s}$-Cys$^{7}$)-Thr$^{8}$-NH$_{2}$
H-D-2-Nal$^{1}$-cyclo(Cys$^{2}$-Tyr$^{3}$-*p*-F-D-Phe$^{4}$-Lys$^{5}$-Val$^{6}$-Cys$^{7}$)-Thr$^{8}$-NH$_{2}$
H-D-2-Nal$^{1}$-cyclo(Cys$^{2}$-Tyr$^{3}$-D-Tyr$^{4}$-Lys$^{5}$-Val$^{6}$-Cys$^{7}$)-Thr$^{8}$-NH$_{2}$
H-D-2-Nal$^{1}$-cyclo(Cys$^{2}$-Tyr$^{3}$-*m*-F-D-Phe$^{4}$-Lys$^{5}$-Val$^{6}$-Cys$^{7}$)-Thr$^{8}$-NH$_{2}$
H-D-2-Nal$^{1}$-cyclo(Cys$^{2}$-Tyr$^{3}$-*o*-F-D-Phe$^{4}$-Lys$^{5}$-Val$^{6}$-Cys$^{7}$)-Thr$^{8}$-NH$_{2}$
H-D-2-Nal$^{1}$-cyclo(Cys$^{2}$-Tyr$^{3}$-*3,5*-diF-D-Phe$^{4}$-Lys$^{5}$-Val$^{6}$-Cys$^{7}$)-Thr$^{8}$-NH$_{2}$
H-D-2-Nal$^{1}$-cyclo(Cys$^{2}$-Tyr$^{3}$-*m*-Br-D-Phe$^{4}$-Lys$^{5}$-Val$^{6}$-Cys$^{7}$)-Thr$^{8}$-NH$_{2}$
H-D-2-Nal$^{1}$-cyclo(Cys$^{2}$-Tyr$^{3}$-*p*-Nitro-D-Phe$^{4}$-Lys$^{5}$-Val$^{6}$-Cys$^{7}$)-Thr$^{8}$-NH$_{2}$
H-D-2-Nal$^{1}$-cyclo(Cys$^{2}$-Tyr$^{3}$-*p*-(2-(2-méthoxyéthoxy)éthoxy)-D-Phe$^{4}$-Lys$^{5}$-Val$^{6}$-Cys$^{7}$)-Thr$^{8}$-NH$_{2}$.

Plus préférentiellement également, le composé selon l'invention est choisi parmi :

H-D-2-Nal'-cyclo(Cys$^{2}$-Tyr$^{3}$-D-Ala$^{4}$-Lys$^{S}$-Val$^{s}$-Cys')-Thr$^{B}$-NH$_{2}$
H-D-2-Nal$^{1}$-cyclo(Cys$^{2}$-Tyr$^{3}$-Ph-D-Gly$^{4}$-Lys$^{5}$-Val$^{6}$-Cys$^{7}$)-Thr$^{8}$-NH$_{2}$;
ou parmi :
H-D-2-Nal$^{1}$-cyclo(Cys$^{2}$-Tyr$^{3}$-D-Val$^{4}$-Lys$^{5}$-Val$^{6}$-Cys$^{7}$)-Thr$^{8}$-NH$_{2}$
H-D-2-Nal$^{1}$-cyclo(Cys$^{2}$-Tyr3-β-(2-naphthyl)-D-Ala$^{4}$-Lys$^{5}$-Val$^{6}$-Cys$^{7}$)-Thr$^{8}$-NH$_{2}$
H-D-2-Nal$^{1}$-cyclo(Cys$^{2}$-Tyr3-β-(9-anthryl)-D-Ala$^{4}$-Lys$^{5}$-Val$^{6}$-Cys$^{7}$)-Thr$^{8}$-NH$_{2}$
H-D-2-Nal$^{1}$-cyclo(Cys$^{2}$-Tyr$^{3}$-homo-D-Phe$^{4}$-Lys$^{5}$-Val$^{6}$-Cys$^{7}$)-Thr$^{8}$-NH$_{2}$
H-D-2-Nal$^{1}$-cyclo(Cys$^{2}$-Tyr$^{3}$-*p*-Ph-D-Phe$^{4}$-Lys$^{5}$-Val$^{6}$-Cys$^{7}$)-Thr$^{8}$-NH$_{2}$.

Préférentiellement également, le composé selon l'invention est choisi parmi :

H-D-2-Nal$^{1}$-cyclo(Cys$^{2}$-Tyr$^{3}$-*p*-(2-(2-méthoxyéthoxy)éthoxy)-D-Phe$^{4}$-Lys$^{5}$-Val$^{6}$-Cys$^{7}$)-Thr$^{8}$-NH$_{2}$
H-2-Nal$^{1}$-cyclo(D-Cys$^{2}$-D-Tyr$^{3}$-Trp$^{4}$-D-Lys$^{5}$-D-Val$^{6}$-D-Cys$^{7}$)-D-Thr$^{8}$-NH$_{2}$.

L'invention a également pour objet un médicament comprenant un composé selon l'invention tel que défini précédemment.

L'invention a également pour objet une composition pharmaceutique comprenant un composé selon l'invention tel que défini précédemment et plus particulièrement lorsque le composé est utilisé à titre de principe actif.

L'invention concerne enfin l'utilisation d'un composé tel que défini précédemment pour fabriquer un médicament ; et préférentiellement un médicament dans lequel un (ou plusieurs) récepteur(s) à la somatostatine est (sont) impliqué(s).

A moins qu'ils ne soient définis d'une autre manière, tous les termes techniques et scientifiques utilisés dans la présente demande ont la même signification que celle couramment comprise par un spécialiste ordinaire du domaine auquel appartient l'invention.

La partie expérimentale suivante est présentée pour illustrer les procédures ci-dessus et ne doit en aucun cas

être considérée comme une limite à la portée de l'invention.

## PARTIE EXPERIMENTALE

### 1. Description des synthèses

#### 1.1 Matériel utilisé

HPLC-MS :

Le système est de marque Waters (2525) avec dégazeur en ligne et système d'injection automatisé (2767). L'élution consiste en un gradient d'eau et d'acétonitrile, avec 0,1 % d'acide formique. La détection des espèces éluées se fait par une barrette de diodes (2996), un détecteur évaporatif à diffusion de lumière (DEDL) et un spectromètre de masse (voir ci-après). La colonne est de type phase inverse, greffée C18, modèle X-Bridge 100 x 4.6 mm avec une taille de particules de 3,5 μm et une taille de pores de 13,5 nm. Le débit est réglé à 1 ml min-1 et le volume d'injection à 20 μl.
Le spectromètre de masse est un Micromass ZQ de marque Waters. L'ionisation se fait par électrospray, avec une température de la source de 120 °C et une tension de cône de 20 V. L'échantillon est introduit de façon continue à 0,3 ml min-1. L'analyseur est de type quadripôle (modèle ZQ2000). Les spectres sont enregistrés à l'aide du logiciel Mass Lynx 4.0 dans le domaine de m/z 100-1000 pour les molécules organiques et 100-2000 pour les peptides.

HPLC-préparative :

Deux systèmes sont utilisés pour la purification de peptides. Le système précédemment décrit équipé d'une colonne type phase inverse, greffée C18, modèle X-Bridge 150 x 19 mm avec une taille de particules de 5 μm et une taille de pores de 13,5 nm. Le débit est de 17 ml min-1. Le deuxième système est un Waters 2545 similaire au précédent, non équipé d'un spectromètre de masse. La colonne est une Thermo Hypurity, de type phase inverse (greffée C18) de taille 21,2 x 250 mm. Elle est éluée par un mélange d'eau et d'acétonitrile avec 0,1 % de TFA à un débit de 20 ml min-1. Les deux systèmes de HPLC préparative sont utilisés en mode isocratique après détermination des conditions optimales.

Analyses RMN :

Les analyses par Résonance Magnétique Nucléaire sont réalisées sur un spectromètre Bruker Advance 400 Ultrashield. Les fréquences d'analyse sont de 400 MHz pour le proton, 376,4 MHz pour le fluor 19 et 100 MHz pour le carbone 13. Les spectres de RMN du fluor sont enregistrés avec une séquence d'une seule impulsion de 90 ° et de durée 19,5 μs. La taille de fenêtre est de 7,5 kHz, le temps de relaxation de 2 s et le temps d'acquisition de 0,87 s. Seize balayages sont réalisés à chaque analyse. Les spectres sont enregistrés à température ambiante, les déplacements chimiques sont exprimés en ppm et les constantes de couplage en Hz. La multiplicité est donnée de la façon suivante : s = singulet, ls = large singulet, d = doublet, ld = large doublet, dd = doublet de doublets, ddd = doublet de doublets dédoublés, t = triplet, lt= large triplet, q = quadruplet, qd = quadruplet dédoublé, m = multiplet, M = massif.

Analyses HRMS :

Les mesures de masse exacte ont été effectuées sur un spectromètre de masse à temps de vol (LCT de Micromass®, UK), muni d'une source electrospray (source Z-spray) en mode positif. La référence externe permettant la mesure de masse exacte est introduite en parallèle de l'échantillon et de façon continue (configuration Lockspray™). Celle ici utilisée est la Leucine Enképhaline qui donne un ion [M+Na]+ à m/z = 578,2591. La résolution de cet appareil est de 6500 et les résultats sont donnés avec un écart par rapport à la masse théorique inférieur à 5 mDa. L'appareil est piloté par le logiciel Masslynx 4.0® L'échantillon solubilisé dans l'eau est injecté dans un flux 50% eau-50% méthanol via une HPLC munie d'un passeur automatique d'échantillons (Alliance 2795 de Waters®, UK) à un débit de 200 μl min-1. Le volume d'injection est de 10 μl. La tension du capillaire est de 2800 V. La tension de cône est de 40 V. La température de la source est de 120°C. La température de désolvatation est de 250°C. Le débit du gaz de désolvatation (azote) est de 500 l h-1. Le débit du gaz de cône (azote) est de 20 l h-1. TDC Stop : 100 mV

Spectrométrie IR :

Les spectres infrarouges des peptides sont enregistrés par réflexion totale atténuée et par transformée de Fourier. L'appareil est un Bruker IFS 66 équipé d'un module 45°N Znse ATR, purgé en continu avec de l'azote. 10 μL

de solution sont déposés sur le cristal et trente balayages sont moyennés à une résolution de 4 cm-1. Le signal de l'eau est soustrait du spectre brut grâce au logiciel OPUS 4.2.

Lyophilisateur :

Le lyophilisateur utilisé est un Christ Alpha 2-4 LD plus connecté à une pompe à palette permettant d'atteindre des vides d'environ 15 μbar. Les échantillons aqueux sont solidifiés dans l'azote liquide avant d'être connectés à cet appareil.

Microscopie :

MET de type Microscope Phillips CM-20 opérant à 200 kV, et MEB de type Léo-Gémini, field emission gun (canon à émission de champ).

## 1.2 Réactifs utilisés

La résine de synthèse peptidique est obtenue chez Novabiochem, division de Merk Bioscience (Schwalbach, Allemagne). La résine échangeuse d'ions provient des laboratoires Bio-Rad (Hercules, Etats-Unis).
L'eau utilisée est doublement désionisée par utilisation d'un système échangeur Milli-Q Plus de Millipore (Billerica, Etats-Unis). Les solvants pour les synthèses et pour les purifications sont achetés chez Aldrich et VWR (West Chester, Etats-Unis) et, sauf mention contraire, sont utilisés sans purification.
Les acide aminés sont achetés chez Bachem (Weil am Rhein, Allemagne), Fluka (Buchs, Suisse), Acros Organics (Geel, Belgique) et NeoMPS (Strasbourg, France).
Acides aminés précurseurs disponibles commercialement :

Fmoc-L-Cys(Trt)-OH, Fmoc-L-Tyr(tBu)-OH, Fmoc-L-Lys(Boc)-OH, Fmoc-L-Val-OH, Fmoc-L-Thr(tBu)-OH, Boc-β-(2-naphthyl)-D-Ala-OH ;

Fmoc-β-(3-benzothienyl)-D-Ala-OH, Fmoc-β-(2-thienyl)-D-Ala-OH, Fmoc-D-His(Boc)-OH, Fmoc-D-Ala-OH, Fmoc-D-Val-OH, Fmoc-β-(1-naphthyl)-D-Ala-OH, Fmoc-β-(2-naphthyl)-D-Ala-OH, Fmoc-β-(9-anthryl)-D-Ala-OH, Fmoc-Ph-D-Gly-OH, Fmoc-homo-D-Phe-OH, Fmoc-*p*-Br-D-Phe-OH, Fmoc-*p*-F-D-Phe-OH, Fmoc-D-Tyr(tBu)-OH, Fmoc-m-F-D-Phe-OH, Fmoc-*o*-F-D-Phe-OH, Fmoc-3,5-diF-D-Phe-OH, Fmoc-m-Br-D-Phe-OH, Fmoc-o-Br-D-Phe-OH, Fmoc-*p*-Nitro-D-Phe-OH, Fmoc-*p*-Ph-D-Phe-OH ;

Fmoc-β-(2-naphtyl)-L-Ala-OH, Fmoc-D-Cys(Trt)-OH, Fmoc-L-Trp(Boc)-OH, Fmoc-D-Lys(Boc)-OH, Fmoc-D-Thr (tBu)-OH.

Synthèse de l'acide aminé Fmoc-β-(2-fluorenyl)-D-Ala-OH

*a/ 2-(benzyloxycarbonylamino)-3-(9H-fluorèn-2-yl)acrylate de méthyle*

À une solution à 0 °C de 2-(benzyloxycarbonylamino)-2-(dimethoxyphosphoryl)acetate de méthyle (2 g, 6,04 mmol, 1 eq) dans du dichlorométhane (60 ml) est ajouté du 1,8-diazabicyclo[5.4.0]undec-7-ene (DBU, 1,35 ml, 1,5 eq). Après 5 min d'agitation le 9H-fluorene-2-carbaldehyde (1,17 mg, 6,04 mmol, 1 eq) est ajouté. Le mélange est laissé 2 h à 0°C sous agitation, puis le bain de glace est retiré, autorisant la température à remonter à une température comprise entre 20 et 30°C. La réaction est laissée sous agitation pendant 2 h. Au milieu réactionnel sont ajoutés 40 mL de dichlorométhane et après lavage par HCl 0,5 M, la phase organique est séchée par du sulfate de sodium, puis évaporée. Une trituration est effectuée grâce à de l'éther diéthylique froid. Le surnageant est récupéré et l'opération est réalisée plusieurs fois. Le solide obtenu est purifié sur gel de silice (Eluant : Hexane-AcOEt 75:25) pour obtenir 1,75 g de produit pur (configuration indéterminée) R = 74 %. L'autre isomère est aussi récupéré. Le rapport des deux isomères est de 95/5.
1H-RMN ($CDCl_3$) : δ 3.84 (M, 2H, fluorène + 3H méthyle); 5,13 (s, 2H, benzyle); 7,25-7,40 (M, 7H), 7,45 (s, 1H); 7,55 (d, J = 7,6, 2H); 7,72 (M, 2H), 7,79 (d, J = 7,6, 1H).
13C-RMN ($CDCl_3$) : δ 36,95; 52,76; 67,63; 120,04; 120,44; 123,36; 125,22; 127,04; 127,52; 128,38, 128,59; 129,17; 132,16; 132,73; 136,07; 141,04; 143,31; 143,52; 144,0; 166,02.
MS (ESI) : m/z 400,0 [M+H]+
IR: λ. (cm-1): 3262, 3034, 2951 (CH alkyle), 1725 (CO), 1699. 1509, 1234, 730.
Point de fusion : 109°C

*b/ (R)-2-(benzyloxycarbonylamino)-3-(9H-fluorèn-2-yl)propanoate de méthyle*

Dans un réacteur métallique fermé, le catalyseur asymétrique au rhodium (35 mg, 2 % mol) est ajouté à une solution de 2-(benzyloxycarbonylamino)-3-(9H-fluorèn-2-yl)acrylate de méthyle (1 g, 2,51 mmol) dans le MeOH (150 ml). Le milieu est purgé de son air et mis sous atmosphère d'hydrogène (50 bars). Après 24 h, le milieu est concentré pour donner quantitativement le produit de réduction.
1H-RMN (CDCl3) : δ 3,15 (dd, J = 14,0, J =6,4, 1H); 3,22 (dd, J = 14,0, J = 6,0, 1H); 3,73 (s, 3H méthyle); 3,85 (s, 2H fluorène); 4,70 (m, 1H); 5,07 (d, J = 12,4, 1H benzyle); 5,12 (d, J = 12,4, 1H, benzyle); 5,25 (1H, NH); 7,11 (d,, J =7,6, 1H); 7,27-7,38 (M, 8H); 7,53 (d, J = 7,6, 2H); 7,58 (d, J = 7,8, 1H); 7,75 (d, J = 7,6, 1H).
13C-RMN ($CDCl_3$): δ 36,74; 38,38; 52,27; 54,95; 66,91; 119,76; 119,85; 124,96; 125,87; 126,64, 126,70, 127,79, 128,04; 128,11, 128,45, 134,14; 136,17; 140,74; 141,27; 143,12; 143,64; 155,58, 172,01.
MS (ESI) m/z : 402,0 [M+H]+
IR: λ. (cm-1): 3347, 3025. 2949 (CH alkyle), 1741 (CO), 1689, 1523, 1256, 1024, 740
Point de fusion : 125 ˚C
Excès énantiomérique déterminé par HPLC chirale : 93,4 %

*c/ acide (R)-2-(benzyloxycarbonylamino)-3-(9H-fluorèn-2-yl)propanoïque*

A une solution de (R)-2-(benzyloxycarbonylamino)-3-(9H-fluorèn-2-yl)propanoate de méthyle (800 mg, 1,99 mmol) dans du dioxane (35 ml) à 0˚C est ajoutée une solution de LiOH 96 mg dans de l'eau (15 ml). La réaction est suivie par CCM (hexane-AcOEt 1:1) (l'acide formé ne migre pas). La réaction est terminée au bout d'une heure. Le milieu est acidifié par HC1 2 M et le produit est extrait par de l'acétate d'éthyle. Le produit est récupéré de manière quantitative (735 mg) après séchage par du sulfate de sodium, évaporation sous vide et cristallisation dans l'éther.
1H-RMN ($(CD_3)_2SO$): δ 2,90 (dd, J = 14,0, J = 9,6, 1H); 3,20 (dd, J = 13,6, J = 4,8, 1H); 3,71 (s, 2H, fluorène); 4,40 (dd, J = 9,6, J = 4,8, 1H); 4,87 (d, J =12,6, 1H benzyle); 4,96 (d, J =12,6, 1H, benzyle); 7,12 (M, 6H); 7,18 (t, J = 7,6, 1H); 7,26 (t, J = 7,6, 1H); 7,32 (s, 1H); 7,43 (d, J = 7,6, 1H); 7,60 (d, J = 7,6, 1H); 7,68 (d, J = 7,8, 1H).
13C-RMN ($(CD_3)_2SO$): δ 36,17; 36,60; 55,68; 65,14; 119,59; 119,71; 125,01; 125,77; 126,43; 126,63; 127,40; 127,59; 127,70; 128,45; 128,15; 136,57; 136,89; 139,39; 140,90; 142,82; 142,88; 155,92; 173,27.
MS (ESI) m/z: 388 [M+H]+
IR: λ. (cm-1): 3333; 3035; 2902 (CH alkyle); 1724 (CO); 1705; 1689; 1531; 1245; 1062; 734.
Point de fusion: 141 ˚C

*d/ acide (R)-2-amino-3-(9H-fluorèn-2-yl)propanoïque*

A une solution d'acide (R)-2-(benzyloxycarbonylamino)-3-(9H-fluorèn-2-yl)propanoïque (650 mg, 1,68 mmol) dans du méthanol (80 mL) est ajouté du Pd/C (65 mg). Le milieu est purgé par de l'azote puis par de l'hydrogène. La réaction est laissée 6 h sous agitation vigoureuse : un précipité apparaît. Le milieu est filtré : un mélange du produit voulu et de Pd/C est récupéré avant solubilisation dans un mélange dioxane-eau (1:1) puis acidification par HCl 2 M de façon à dissoudre l'acide aminé. Cette solution est filtrée, le dioxane est évaporé sous vide puis la phase aqueuse est neutralisée par ajout de NaOH 2 M. Après évaporation, la poudre blanche obtenue est lavée par de l'eau de façon à enlever les sels en excès. 500 mg de l'aminoacide sont récupérés. Le rendement est de 85 %.
MS (ESI) m/z : 254 [M+H]+
IR: λ. (cm-1): 3425; 3019; 2960 (CH alkyle); 1567; 1402; 1316; 737.
Point de fusion : 225 ˚C
UV λ. (nm): 206 (abs = 0,868), 267 (abs = 0,446), 303 (abs = 0,203)
Fluorescence : (excitation à 267 nm) maximum à 312 nm.
Excès énantiomérique déterminé par HPLC chirale : 93,4 %.

*e/ acide (R)-2-(((9H-fluorèn-9-yl)méthoxy)carbonylamino)-amino-3-(9H-fluorèn-2-yl) propanoïque ou Fmoc-β-(2-fluorenyl)-D-Ala-OH*

A une solution de l'aminoacide acide (R)-2-amino-3-(9H-fluorèn-2-yl)propanoïque (560 mg. 2,21 mmol, 1 eq.) dans un mélange dioxane (50 mL)-carbonate de sodium à 10 % dans l'eau (30 ml) est ajoutée goutte à goutte une solution de FmocOSu (820 mg, 2,43 mmol, 1,1 eq) dans 20 ml de dioxane. La disparition de FmocOSu est suivie par CCM éluant hexane-AcOEt (1:1). La réaction est agitée pendant 3h. Le mélange réactionnel est extrait par de l'acétate d'éthyle et la phase organique est récupérée et évaporée. Le solide obtenu est cristallisé dans AcOEt avant filtration. 860 mg de produit attendu sont récupérés. R = 82%.
1H-RMN ($(CD_3)_2SO$): δ 3,00 (dd, J = 13,2, J = 5,8, 1H); 3,13 (dd, J = 13,2, J = 4,9, 1H); 3,76 (s, 2H, fluorène); 3,81 (dd,

J = 6,4, J = 4,8, 1H), 4,12 (dd, J = 9,8, J = 6,4, 1H, CHβ Fmoc); 4,19 (t, J = 6,4, 1H, Fmoc); 4,30 (dd, J = 9,8, J = 6,4, 1H, CHβ Fmoc); 6,35 (s, 1H, NH); 7,12 (d, J = 7,6, 1H); 7,20-7,40 (M, 7H); 7,53 (d, J = 7,4, 1H); 7,62 (t, J = 7,2, 2H); 7,67 (d, J = 7,8, 1H); 7,81 (d, J = 7,6, 1H); 7,7 (d, J = 7,6, 2H).
13C-RMN (CD3SO): la molécule est dégradée.
MS (ESI) m/z : 476,0 [M+H]+
IR: λ. (cm-1): 3382. 3051; 2958 (CH alkyle); 1678; 1605; 1528. 1411; 1254 ; 1037; 735.

Synthèse de la N-fluorènyiméthoxycarbonyl-4-(2-(2-méthoxyéthoxy)éthoxy)-D-Phe ou Fmoc-*p*-O-2-(2-méthoxyéthoxy)éthoxy-D-Phe

*a/ N-terbutyloxycarbonyl-4-(2-(2-méthoxyéthoxy)éthoxy)-D-Phe*

100 mg (0,35 mmol) de Boc-D-tyrosine-OH ainsi que 155 mg (1,12 mmol, 3,1 eq) sont introduits dans 1 ml de DMF et 100 μl de $H_2O$. A 0 °C, 180 μl (1,43 mmol, 4,1 eq) de 1-bromo-2-(2-méthoxyéthoxy)éthane sont introduits et la réaction est mise à chauffer à 50°C pendant 7 h. Le milieu réactionnel est alors dissous dans l'acétate d'éthyle et de l'acide chlorhydrique dilué est ajouté afin d'amener le pH de la phase aqueuse à 2. La phase organique est lavée avec de l'acide chlorhydrique dilué, puis séchée sur du sulfate de magnésium. Après évaporation, on recueille 218,9 mg de solide amorphe. Ce solide est dissous dans 2 ml de soude 1 M et 2 ml de dioxane et laissé sous agitation 1 h 30. Le pH est ajusté à 2 avec de l'acide chlorhydrique dilué, puis le produit est extrait par l'acétate d'éthyle. On recueille 137,7 mg de produit brut. La purification est réalisée par colonne de silice avec un éluant acétate d'éthyle-cyclohexane-acide acétique 60:40:1 %. Après évaporation on recueille 94,6 mg de produit pur, soit un rendement de 68 %.
RMN $^1H$ (400 MHz, $CDCl_3$) : δ 1,41 (s, 9H, *t*Bu); 3,01 (dd, J = 13,7, J = 5,7, 1H, CHβ); 3,11 (dd, J = 13,7, J = 4,8, 1H, CHβ); 3,39 (s, 3H, $OCH_3$); 3,55-3,60 (M, 2H, $CH_2$ éthoxy); 3,69-3,74 (M, 2H, $CH_2$ éthoxy); 3,81-3,87 (M, 2H, $CH_2$ éthoxy); 4,07-4,16 (M, 2H, $CH_2$ éthoxy); 4,50-4,59 (m, 1H CHα); 4,97 (d, J = 7,7, 1H, NH); 6,84 (d, J = 8,3, 2H, CH Ar); 7,07 (d, J = 8,3, 2H, CH Ar).
MS (ESI) m/z : 384,2 $[M+H]^+$

*b/ 4-(2-(2-méthoxyéthoxy)éthoxy)-D-Phe*

1.89 g de N-*ter*-butyloxycarbonyl-4-(2-(2-méthoxyéthoxy)éthoxy)-D-Phe sont introduits dans 26 ml de HCl 35 % et 74 ml d'acétate d'éthyle. La réaction est laissée 3 h sous agitation. Après évaporation, le solide est trituré trois fois par de l'éther diéthylique pour obtenir 1,12 g de solide blanc, soit un rendement de 66 %.
RMN $^1H$ ($(CD_3)_2SO$) : δ 3,04 (d, J = 6,3, 2H, CHHβ); 3,23 (s, 3H, $OCH_3$); 3,41-3,46 (M, 2H, $CH_2$ éthoxy); 3,53-3,59 (M, 2H, $CH_2$ éthoxy); 3,68-3,73 (M, 2H, $CH_2$ éthoxy); 4,01-4,06 (m, 2H, $CH_2$ éthoxy); 4,09-4,12 (m, 1H CHα); 6,89 (d, J = 8,8, 1H, CH Ar); 7,16 (d, J = 8,8, 1H, CH Ar).
RMN $^{13}C$ ($(CD_3)_2SO$) : δ 34,84 (Cβ); 53,17 (Cα); 58,11 ($CH_3O$); 67,16 ($CH_2$ éthoxy); 68,97 ($CH_2$ éthoxy); 68,77 ($CH_2$ éthoxy); 71,34 ($CH_2$ éthoxy); 114,57 (C *meta* Ar); 126,61 (C *ortho* ar); 130,65 ($CH_2$-$\underline{C}$ Ar); 157,84 (O-C Ar); 170,30 (CO).
MS (ESI) m/z : 284, 1 $[M+H]^+$

*c/ N-fluorènylméthoxycarbonyl-4-(2-(2-méthoxy)éthoxy)-D-Phe*

812 mg (2,87 mmol) de 4-(2-(2-méthoxyéthoxy)éthoxy)-D-Phe sont introduits dans 4 ml d'eau distillée et 4 ml d'acétone. On y ajoute 300 mg (2,83 mmol, 0,98 eq) de $Na_2CO_3$ et 954,6 mg (2,83 mmol, 0,98 eq) de FmocOSu. La suspension blanche est agitée 5 h 30 à une température comprise entre 20 et 30°C. L'acétone est alors évaporée, le milieu ajusté à pH = 2 par de l'acide chlorhydrique et le produit est extrait par l'acétate d'éthyle. Les phases organiques sont rassemblées, lavées par une solution de NaCl saturée puis séchées sur $MgSO_4$ anhydre. Après évaporation, on obtient 1,5 g de produit brut. Ce produit est purifié sur 120 g environ de silice, avec un éluant dichlorométhane-méthanol-acide acétique 98:2:5, pour obtenir 939 mg de solide blanc, soit un rendement de 65 %.
RMN $^1H$ ($CDCl_3$) : δ 3,05 (dd, J = 14,0, J = 5,7, 1H, CHβ); 3,13 (dd, J = 14,0, J = 5,4, 1H, CH'β); 3,38 (s, 3H, $OCH_3$); 3,55-3,59 (M, 2H, $CH_2$ éthoxy); 3,67-3,72 (M, 2H, $CH_2$ éthoxy); 3,80-3,85 (M, 2H, $CH_2$ éthoxy); 4,04-4,10 (M, 2H, $CH_2$ éthoxy); 4,20 (t, *J* = 6,8, 1H, CH); 4,34 (dd, J = 10,8, J = 6,8, 1H, CHβ Fmoc); 4,44 (dd, J = 10,8, J = 6,8, 1H, CH'β Fmoc); 4,63 (dd, J = 14,0, J = 5,7, 1H, CHα); 5,30 (s, 1H, NH); 6,81 (d, J = 8,1, 2H, CH ar,); 7,03 (d, J = 8,1, 2H, CH Ar,); 7,30 (t, J = 7,4, 2H, fluorènyle); 7,40 (t, J = 7,4, 2H, fluorènyle); 7,56 ( t, J = 5,8, 2H, fluorènyle); 7,76 (d, J = 7,4, fluorènyle).
RMN $^{13}C$ ($CDCl_3$) : δ 36,89 ($CH_2\beta$); 47,08 (CH Fmoc); 54,66 (CHα); 58,96 ($CH_3O$); 66,96 ($CH_2$ Fmoc); 67,22 ($CH_2$ éthoxy); 69,68 ($CH_2$ éthoxy); 70,55 ($CH_2$ éthoxy); 71,83 ($CH_2$ éthoxy); 114,65 (C *meta* Phe,); 119,92 (CH fluorènyle); 125,01 (CH fluorènyle); 127,02 (C *ortho* ar); 127,67 (CH fluorényle); 127,77 (CH fluorènyle); 130,34 ($CH_2$-$\underline{C}$ Phe,);

141,24 (C fluorènyle); 143,64 (C fluorènyle); 155,70 (CO Fmoc); 157,84 (O-C ar,); 170,30 (CO). MS (ESI) m/z : 506,0 $[M+H]^+$

## 1.3 Préparation des composés octapeptidiques

### *1.3.1 Procédure générale pour la synthèse de peptides*

La synthèse comporte 4 étapes principales comme le montre le schéma 1 :

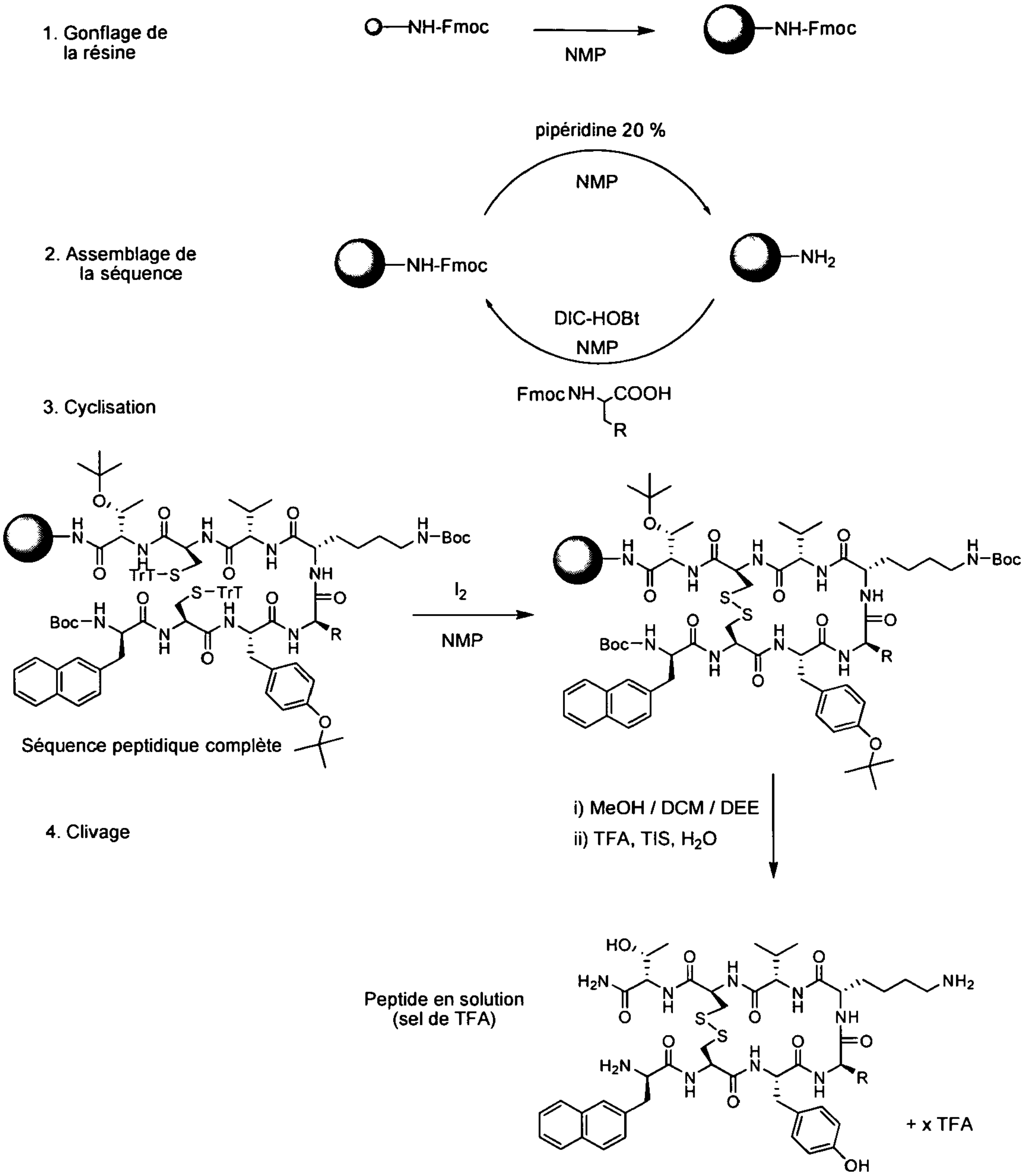


Schéma 1

1/ Mouillage de la résine :

La résine 4-(2',4'-diméthoxyphényl-fluorènylméthoxycarbonyl-aminométhyl)-phénoxyacétamido-norleucyl-(4-méthyl)-benzhydrylamine - base polystyrène - divinylbenzène (Rink Amide MBHA) est introduite dans une seringue munie d'un verre fritté, d'un robinet à une extrémité et d'un bouchon à l'autre. On la remplit avec de la NMP et le mélange est mis sous agitation douce pendant 1 h. Le solvant est ensuite éliminé par filtration.

2/ Couplage des acides aminés :

Les acides aminés sont couplés les uns aux autres dans l'ordre souhaité au moyen d'une réaction de couplage. L'acide aminé (2 eq) est introduit avec le 1-hydroxybenzotriazole (HOBt, 2,2 eq) et le *N*,*N*-Diisopropylcarbodiimide (DIC, 2,2eq) dans la *N*-méthylpyrrolidinone (NMP, 5 ml/g de résine) dans un tube à essai et agité quelques minutes. Il est ensuite mis en présence de la résine dans son récipient. Le mélange réactionnel est agité pendant 1 h 30 puis filtré. La technique du double couplage est utilisée : le mélange réactionnel est filtré quand la réaction est environ à 50 % d'avancement, et des réactifs frais sont réintroduits, afin d'optimiser la vitesse de réaction et la pureté du produit final. La deuxième étape consiste à déprotéger le nouvel acide aminé introduit, afin de permettre un nouveau couplage. La déprotection est réalisée par trois traitements par de la pipéridine dans la NMP (20 % v/v), 5 ml/g de résine suivis par trois lavages à la NMP (10 ml/g de résine). Afin de suivre la réaction, 5 μl du filtrat correspondant au premier traitement, puis 10 μl des deux suivants, ainsi que le premier lavage, soit 4 échantillons, sont introduits dans 2 ml de pipéridine avant mesure de l'absorbance UV à 290 nm. Entre chaque étape, on procède à trois lavages de la résine par la NMP (10 ml/g de résine). Cette étape d'assemblage consiste donc en deux réactions : la réaction de couplage d'acides aminés et la réaction de déprotection du groupement Fmoc, à réaliser itérativement jusqu'à achèvement de la séquence peptidique.

3/ Formation du pont disulfure :

Une fois la séquence assemblée, le peptide doit être cyclisé par formation du pont disulfure. Le pont disulfure est formé par trois traitements de diiode 1 eq dans la NMP (5 ml/g de résine) pendant respectivement 2 min, 3 min et 5 min. La résine est ensuite lavée 5 fois par du DCM et 5 fois par de la NMP afin d'éliminer l'excès d'iode retenu dans la résine (10 ml/g de résine).

4/ Clivage :

La résine doit être préparée au clivage par deux lavages à la NMP, deux lavages au méthanol (MeOH), deux lavages au dichlorométhane (DCM) et deux lavages à l'éther diéthylique (DEE) (10 ml par gramme de résine). La résine est ensuite mise sous vide pendant une journée. Le clivage se fait dans un ballon en verre muni d'un agitateur magnétique. Le mélange réactionnel est formé d'acide trifluoroacétique (TFA, 10 ml/g de résine) ainsi que de triisopropylsilane (TIS) et de l'eau (3 % et 2 % v/v). La réaction est agitée 4 h à température ambiante. Le milieu est ensuite filtré sur verre fritté et le solide lavé deux fois par le TFA. Le filtrat est ensuite évaporé pour obtenir un liquide blanc très épais. Celui-ci est dissous dans un mélange eau-acétonitrile 1:1 afin d'être lyophilisé. Après cette étape, le peptide se présente sous forme de sel de trifuoroacétate.

*1.3.2 Purification*

La purification est réalisée par chromatographie liquide haute performance (HPLC) préparative. La phase stationnaire est dite « inverse » car greffée avec des chaînes alkyles $C_{18}$. La phase mobile est constituée d'un mélange fixé (isocratique) d'eau et d'acétonitrile avec 0,1 % de TFA ou 1 % d'acide formique servant à neutraliser les silanols non greffés résiduels pouvant exister sur la phase stationnaire.

Le peptide doit être dissous dans un mélange eau-acétonitrile pour être injecté en HPLC préparative. Une étude de solubilité est d'abord réalisée sur une petite quantité. Elle permet d'établir le pourcentage d'acétonitrile optimal et la quantité maximale de peptide. Un pourcentage d'acétonitrile le plus faible possible avec une concentration de peptide très élevée et une solution résultante limpide constituent des conditions idéales. A titre d'exemple, la solubilité du dérivé 4-para-fluorophénylalanine (exemple 13 ci-après) a été établie à 49 g $l^{-1}$ dans une solution eau-acétonitrile 58:42.

Après purification, les fractions contenant le peptide purifié sont rassemblées et évaporées sous vide. Le peptide pur est alors récupéré dans de grandes quantités de solvant à évaporer avant de procéder à une lyophilisation. Le peptide se présente en général sous forme de sel de trifluoroacétate devant être échangé par un acétate avant analyse physico-chimique.

*1.3.3 Echange d'ions*

L'échange est réalisé sur une résine de type échangeuse d'anions forts (AG1-X8 Biorad). 245 mg de cette résine sont d'abord lavés par trois fois 10 ml d'acide acétique 1,6 N puis par trois fois 10 ml d'acide acétique 0,16 M. 20 mg de peptide en sel de TFA sont alors introduits dans 4 ml d'eau et le récipient est agité de manière rotative pendant 1 h. Le liquide est alors filtré et la résine lavée par deux fois 1 ml d'eau distillée. Les fractions sont rassemblées puis lyophilisées.

**1.4 Exemples**

Les produits ont été caractérisés selon les méthodes classiques connues de l'homme de l'art décrites précédemment.

***Exemple 1 :*** H-D-2-Nal$^1$-cyclo(Cys$^2$-Tyr$^3$-β-(3-benzothiènyl)-D-Ala$^4$-Lys$^5$-Val$^6$-Cys$^7$)-Thr$^8$-$NH_2$•2 $CH_3COOH$

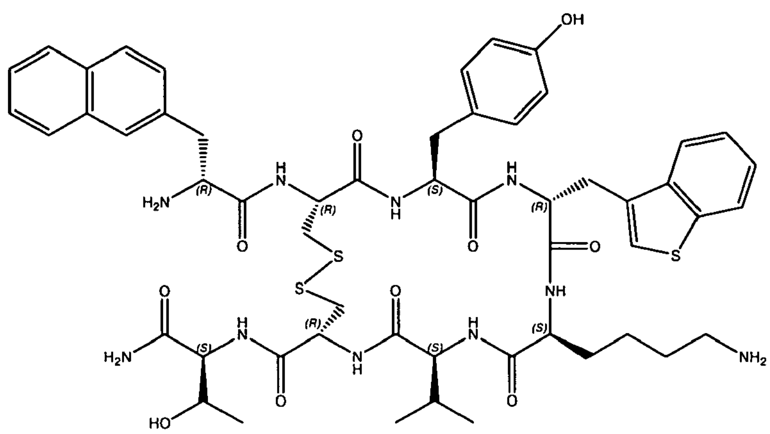

Le peptide ouvert est obtenu selon le protocole décrit précédemment par la succession de couplage, déprotection du groupement Fmoc (9-fluorènylméthyloxycarbonyl) et couplage de l'acide aminé suivant sur support solide dans l'ordre suivant : Fmoc-L-Thr(tBu)-OH puis Fmoc-L-Cys(Trt)-OH puis Fmoc-L-Val-OH puis Fmoc-L-Lys(Boc)-OH puis Fmoc-β-(3-benzothiènyl)-D-Ala-OH puis Fmoc-L-Tyr(tBu)-OH puis Fmoc-L-Cys(Trt)-OH et enfin Boc-β-(2-naphthyl)-D-Ala-OH.

Le composé est cyclisé par la formation d'un pont disulfure, puis clivé de la résine, purifié, et enfin obtenu sous forme d'acétate selon le mode opératoire décrit précédemment pour conduire au composé H-D-2-Nal$^1$-cyclo(Cys$^2$-Tyr$^3$-β-(3-benzothiènyl)-D-Ala$^4$-Lys$^5$-Val$^6$-Cys$^7$)-Thr$^8$-$NH_2$•2 $CH_3COOH$.

HPLC : tr = 9,8 min

$^1$H NMR (400 Mhz, $D_2O$): δ 0,28-0,40 (m, 1H); 0,42-0,54 (m, 1H); 0,90 (d, J = 6,6, 3H); 0,92 (d, J = 6,6, 3H); 1,20 (d, J = 6,4, 3H); 1,28-1,34 (m, 1H); 1,48-1,62 (m, 1H); 1,90 (s, 6H); 1,90-2,20 (m, 1H); 2,44 (dd, J = 14,6, J = 4,2, 1H); 2,57 (dd, J = 14,8, J = 9,3, 1H); 2,65-2,82 (M, 4H); 2,92 (d, J = 7,4, 2H); 3,06 (dd, J = 13,9, J = 5,5, 1H); 3,12-3,22 (m, 1H); 3,29 (dd, J = 13,3, J = 9,5, 1H); 3,43 (dd, J = 13,5, J = 5,5, 1H); 3,81 (dd, J = 10,3, J = 3,7, 1H); 4,24 (dd, J = 6,4, J = 4,1, 1H); 4,26-4,38 (M, 3H); 4,64 (t, J = 7,5, 2H); 6,84 (d, J = 8,2, 2H); 7,12 (d, J = 8,2, 2H); 7,31 (s, 1H); 7,38-7,50 (M, 3H); 7,54 (dd, J = 9,4, J = 3,8, 1H); 7,55 (dd, J = 9,4, J = 3,8, 1H); 7,71 (d, J = 7,7, 1H); 7,78 (s, 1H); 7,85-7,90 (m, 1H); 7,90-7,95 (M, 2H); 7,98 (d, J = 7,5, 1H).

HRMS ($H_2O$) m/z: 1113,4368 [M+H]+ (calc. 1113,4360) C54 H69 N10 O10 S3

***Exemple 2*** : H-D-2-Nal$^1$-cyclo(Cys$^2$-Tyr$^3$-β-(2-thienyl)-D-Ala$^4$-Lys$^5$-Val$^6$-Cys$^7$)-Thr$^8$-$NH_2$•2 $CH_3COOH$

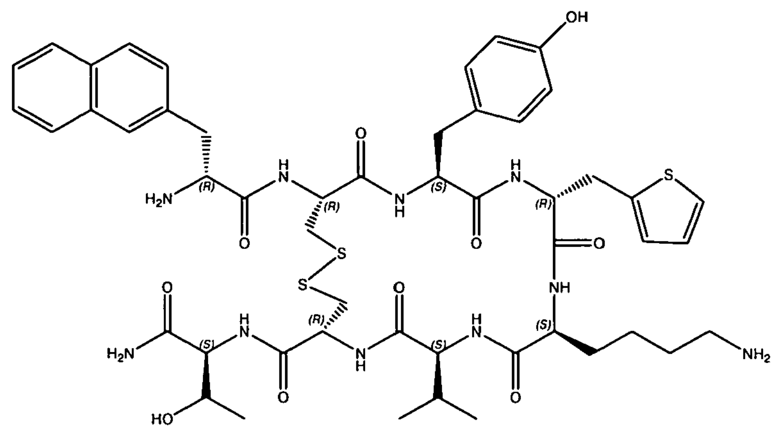

Le peptide ouvert est obtenu selon le protocole décrit précédemment par la succession de déprotection du groupement Fmoc (9-fluorènylméthyloxycarbonyl) et couplage de l'acide aminé suivant sur support solide dans l'ordre suivant : Fmoc-L-Thr(tBu)-OH puis Fmoc-L-Cys(Trt)-OH puis Fmoc-L-Val-OH puis Fmoc-L-Lys(Boc)-OH puis Fmoc-β-thiènyl)-D-Ala-OH puis Fmoc-L-Tyr(tBu)-OH puis Fmoc-L-Cys(Trt)-OH et enfin Boc-β-(2-naphthyl)-D-Ala-OH.

Le composé est cyclisé par la formation d'un pont disulfure, puis clivé de la résine, purifié, et enfin obtenu sous forme d'acétate selon le mode opératoire décrit précédemment pour conduire au composé H-D-2-$Nal^1$-cyclo($Cys^2$-$Tyr^3$-β-(2-thiènyl)-D-$Ala^4$-$Lys^5$-$Val^6$-$Cys^7$)-$Thr^8$-$NH_2$•2 $CH_3COOH$.

HPLC : tr = 9,7 min

$^1$H NMR (400 Mhz, $D_2O$) : δ 0,76-0,88 (m, 1H); 0,92 (d, J = 6,6, 3H); 0,95 (d, J = 6,6, 3H); 1,19 (d, J = 6,4, 3H); 1,40-1,54 (M, 3H); 1,76-1,88 (m, 1H); 1,93 (s, 6H); 2,14-2,26 (m, 1H); 2,41 (dd, J = 14,8, J = 3,8, 1H); 2,63 (dd, J = 15,4, J = 9,7, 1H); 2,68 (dd, J = 15,0, J = 3,6, 1H); 2,74-2,82 (m, 1H); 2,83-2,95 (M, 4H); 3,10 (d, J = 8,0, 1H); 3,37 (dd, J = 13,6, J = 8,6, 1H); 3,47 (dd, J = 13,8, J = 5,6, 1H); 4,02 (d, J = 9,7, 1H); 4,07 (dd, J = 10,8, J = 3,5, 1H); 4,21 (qd, J = 10,4, J = 6,4, 1H); 4,28 (t, J = 8,1, 1H); 4,31 (d, J = 4,0, 1H); 4,42 (dd, J = 8,6, J = 6,2, 1H); 4,62 (t, J = 7,4, 1H); 4,89 (dd, J = 10,5, J = 3,6, 1H); 4,93 (dd, J = 10,5, J = 3,6, 1H); 6,82 (M, 3H); 6,98 (M, 2H); 7,11 (d, J = 8,4, 2H); 7,31 (d, J = 5,1, 1H); 7,47 (d, J = 8,2, 1H); 7,54 (dd, J = 9,5, J = 3,3, 1H); 7,55 (dd, J = 9,5, J = 3,3, 1H); 7,8 (s, 1H); 7,82-7,88 (M, 3H).

HRMS ($H_2O$) m/z : 1063,4230 [M+H]+ (calc. 1063,4204) C50 H67 N10 O10 S3

***Exemple 3*** : H-D-2-$Nal^1$-cyclo($Cys^2$-$Tyr^3$-D-$His^4$-$Lys^5$-$Val^6$-$Cys^7$)-$Thr^8$-$NH_2$•2 $CH_3COOH$

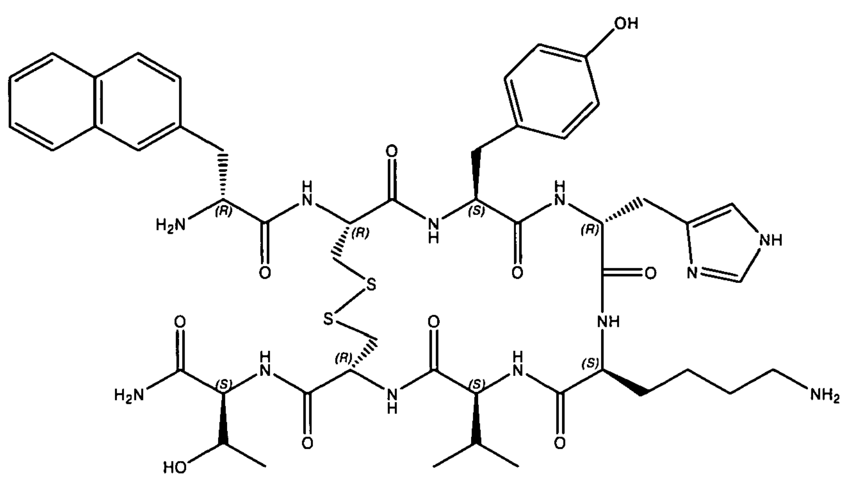

Le peptide ouvert est obtenu selon le protocole décrit précédemment par la succession de déprotection du groupement Fmoc (9-fluorènylméthyloxycarbonyl) et couplage de l'acide aminé suivant sur support solide dans l'ordre

suivant : Fmoc-L-Thr(tBu)-OH puis Fmoc-L-Cys(Trt)-OH puis Fmoc-L-Val-OH puis Fmoc-L-Lys(Boc)-OH puis Fmoc-D-His-OH puis Fmoc-L-Tyr(tBu)-OH puis Fmoc-L-Cys(Trt)-OH et enfin Boc-β-(2-naphthyl)-D-Ala-OH.

Le composé est cyclisé par la formation d'un pont disulfure, puis clivé de la résine, purifié, et enfin obtenu sous forme d'acétate selon le mode opératoire décrit précédemment pour conduire au composé H-D-2-$Nal^1$-cyclo($Cys^2$-$Tyr^3$-D-$His^4$-$Lys^5$-$Val^6$-$Cys^7$)-$Thr^8$-$NH_2$•2 $CH_3COOH$.

HPLC : tr = 6,3 min

$^1$H NMR (400 Mhz, $D_2O$) : δ 0,90 (d, J = 6,6, 3H); 0,94 (d, J = 6,6, 3H); 1,02-1,14 (M, 2H); 1,17 (d, J = 6,6, 3H); 1,51-1,64 (M, 3H); 1,83-1,90 (m, 1H); 1,91 (s, 6H); 2,10-2,18 (m, 1H); 2,53 (dd, J = 14,6, J = 4,4, 1H); 2,60 (dd, J = 14,8, J = 9,5, 1H); 2,70 (dd, J = 14,6, J = 4,4, 1H); 2,75 (dd, J = 14,4, J = 4,2, 1H); 2,84-2,94 (M, 5H); 2,99 (dd, J = 15,2, J = 7,2, 1H); 3,36 (dd, J = 13,9, J = 8,6, 1H); 3,46 (dd, J = 13,8, J = 5,7, 1H); 4,04 (d, J = 9,7, 1H); 4,13-4,23 (M, 2H); 4,26-4,32 (M, 2H); 4,40 (dd, J = 8,7, J = 5,9, 1H); 4,55 (t, J = 7,7, 1H); 4,86 (dd, J = 9,6, J = 4,4, 1H); 4,92 (dd, J = 10,4, J = 4,6, 1H); 6,74 (d, J = 8,6, 2H); 7,01 (d, J = 1,3, 1H); 7,04 (d, J = 8,6, 2H); 7,46 (dd, J = 8,5, J = 1,7, 1H); 7,52 (ddd, J = 6,7, J = 4,4, J = 1,6, 1H); 7,53 (ddd, J = 6,7, J = 4,4, J = 1,6, 1H); 7,80 (s, 1H); 7,93-7,93 (M, 3H); 8,29 (d, J = 1,1; 1H).

HRMS ($H_2O$) m/z: 1047,4507 [M+H]+ (calc. 1047,4545) C49 H66 N12 O10 S2

***Exemple 4*** : H-D-2-$Nal^1$-cyclo($Cys^2$-$Tyr^3$-D-$Ala^4$-$Lys^5$-$Val^6$-$Cys^7$)-$Thr^8$-$NH_2$•2 $CH_3COOH$

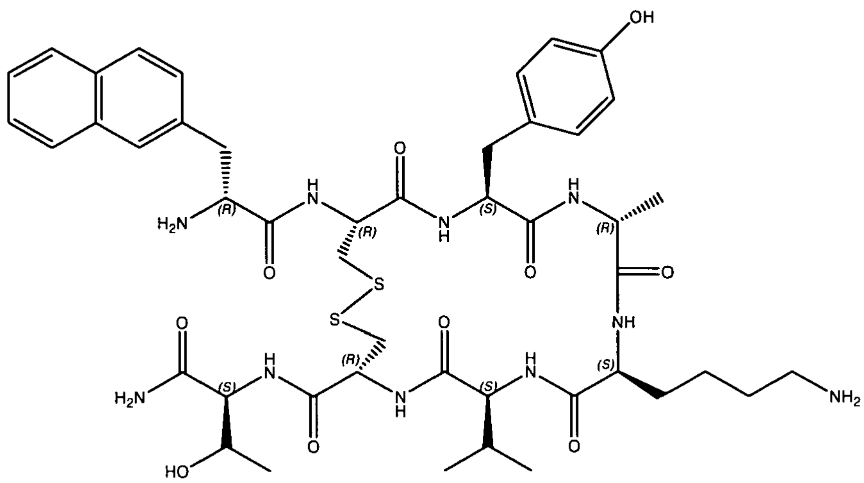

Le peptide ouvert est obtenu selon le protocole décrit précédemment par la succession de déprotection du groupement Fmoc (9-fluorènylméthyloxycarbonyl) et couplage de l'acide aminé suivant sur support solide dans l'ordre suivant : Fmoc-L-Thr(tBu)-OH puis Fmoc-L-Cys(Trt)-OH puis Fmoc-L-Val-OH puis Fmoc-L-Lys(Boc)-OH puis Fmoc-D-Ala-OH puis Fmoc-L-Tyr(tBu)-OH puis Fmoc-L-Cys(Trt)-OH et enfin Boc-β-(2-naphthyl)-D-Ala-OH.

Le composé est cyclisé par la formation d'un pont disulfure, puis clivé de la résine, purifié, et enfin obtenu sous forme d'acétate selon le mode opératoire décrit précédemment pour conduire au composé H-D-2-$Nal^1$-cyclo($Cys^2$-$Tyr^3$-D-$Ala^4$-$Lys^5$-$Val^6$-$Cys^7$)-$Thr^8$-$NH_2$•2 $CH_3COOH$.

HPLC : tr = 9,8 min

$^1$H NMR (400 Mhz, $D_2O$) : δ 0,93 (d, J = 6,6, 3H); 0,96 (d, J = 6,6, 3H); 1,18 (d, J = 7,8, 3H); 1,20 (d, J = 6,6, 3H); 1,34-1,44 (M, 2H); 1,60-1,68 (M, 2H); 1,68-1,80 (M, 2H); 1,90 (s, 6H); 1,92-2,20 (m, 1H); 2,12-2,22 (m, 1H); 2,45 (dd, J = 14,6, J = 4,9, 1H); 2,55 (dd, J = 14,7, J = 8,7, 1H); 2,74 (d, J = 1,3, 1H); 2,76 (s, 1H); 2,90-3,00 (M, 4H); 3,3 (dd, J = 13,5, J = 9,1, 1H); 3,44 (dd, J = 13,7, J = 5,6, 1H); 4,04 (d, J = 9,5, 1H); 4,07 (d, J = 7,3, 1H); 4,2 (dd, J = 9,9, J = 4,1, 1H); 4,24 (dd, J = 6,5, J = 3,9, 1H); 4,28-4,32 (m, 1H); 4,33 (d, J = 4,0, 1H); 4,55 (dd, J = 7,8, J = 7,3, 1H); 4,82-4,88 (m, 1H); 6,82 (d, J = 8,6, 2H); 7,11 (d, J = 8,6, 2H); 7,47 (dd, J = 8,5, J = 1,7, 1H); 7,54 (ddd, J = 5,7, J = 4,7, J = 2,0, 1H); 7,55 (ddd, J = 5,7, J = 4,7, J = 2,0, 1H); 7,79 (d, J = 0,9, 1H); 7,86-7,96 (M, 3H).

HRMS ($H_2O$) m/z : 1003,4161 [M+Na]+ (calc. 1003,4146) C46 H64 Na N10 010 S2

***Exemple 5 :*** H-D-2-$Nal^1$-cyclo($Cys^2$-$Tyr^3$-β-(2-thienyl)-D-$Ala^4$-$Lys^5$-$Val^6$-$Cys^7$)-$Thr^8$-$NH_2$•2 $CH_3COOH$

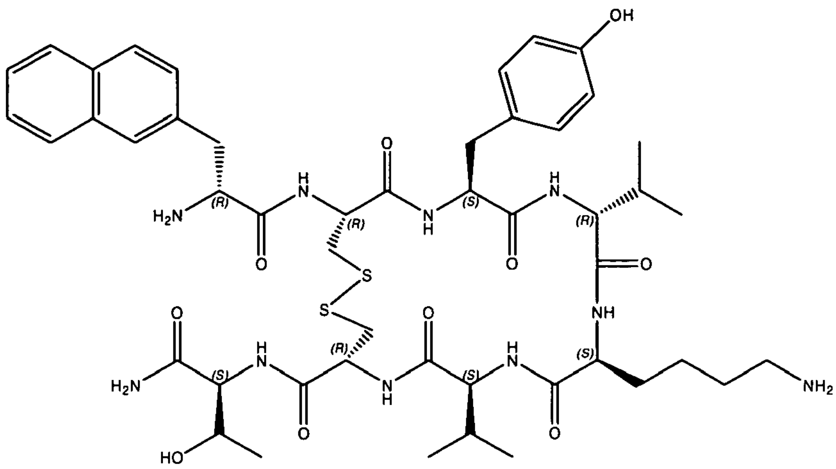

Le peptide ouvert est obtenu selon le protocole décrit précédemment par la succession de déprotection du groupement Fmoc (9-fluorènylméthyloxycarbonyl) et couplage de l'acide aminé suivant sur support solide dans l'ordre suivant : Fmoc-L-Thr(tBu)-OH puis Fmoc-L-Cys(Trt)-OH puis Fmoc-L-Val-OH puis Fmoc-L-Lys(Boc)-OH puis Fmoc-D-Val-OH puis Fmoc-L-Tyr(tBu)-OH puis Fmoc-L-Cys(Trt)-OH et enfin Boc -β-(2-naphthyl)-D-Ala-OH.

Le composé est cyclisé par la formation d'un pont disulfure, puis clivé de la résine, purifié, et enfin obtenu sous forme d'acétate selon le mode opératoire décrit précédemment pour conduire au composé H-D-2-$Nal^1$-cyclo ($Cys^2$-$Tyr^3$-D-$Val^4$-$Lys^5$-$Val^6$-$Cys^7$)-$Thr^8$-$NH_2$•2 $CH_3COOH$.

HPLC : tr = 10,7 min

$^1$H NMR (400 Mhz, $D_2O$) : δ 0,73 (d, J = 6,6, 3H); 0,80 (d, J = 6,8, 3H); 0,93 (d, J = 6,8, 3H); 0,98 (d, J = 6,6, 3H); 1,19 (d, J = 6,6, 3H); 1,30-1,50 (M, 2H); 1,58-1,82 (M, 4H); 1,60-2,08 (M, 4H); 1,92 (s, 6H); 1,96-2,06 (m, 1H); 2,14-2,24 (m, 1H); 2,51 (dd, J = 14,8, J = 3,8, 1H); 2,62 (dd, J = 14,8, J = 10,0, 1H); 2,69 (dd, J = 14,5, J = 3,5, 1H); 2,79 (dd, J = 14,9, J = 11,2, 1H); 2,88-3,00 (M, 3H); 3,37 (dd, J = 13,9, J = 8,6, 1H); 3,57 (dd, J = 13,9, J = 5,8, 1H); 3,60 (d, J = 9,7, 1H); 4,05 (d, J = 9,7, 1H); 4,21 (dd, J = 6,3, J = 3,9, 1H); 4,25 (dd, J = 10,7, J = 3,7, 1H); 4,30 (d, J = 3,8, 1H); 4,42 (dd, J = 8,6, J) = 5,8, 1H); 4,61 (dd, J = 8,4, J = 6,9, 1H); 4,95 (dd, J = 9,3, J = 3,6, 1H); 4,96 (dd, J = 11,8, J = 2,9, 1H); 6,81 (d, J = 8,6, 2H); 7,12 (d, J = 8,4, 2H); 7,47 (dd, J = 8,4, J = 1,5, 1H); 7,54 (dd, J = 10,2, J = 3,8, 1H); 7,55 (dd, J = 10,2, J = 3,8, 1H); 7,80 (s, 1H); 7,84-7,94 (M, 3H).

HRMS ($H_2O$) m/z : 1009,4627 [M+H]+ (calc. 1009,4640) C48 H69 N10 O10 S2

***Exemple 6*** : H-D-2-$Nal^1$-cyclo($Cys^2$-$Tyr^3$-β-(1-naphthyl)-D-$Ala^4$-$Lys^5$-$Val^6$-$Cys^7$)-$Thr^8$-$NH_2$•2 $CH_3COOH$

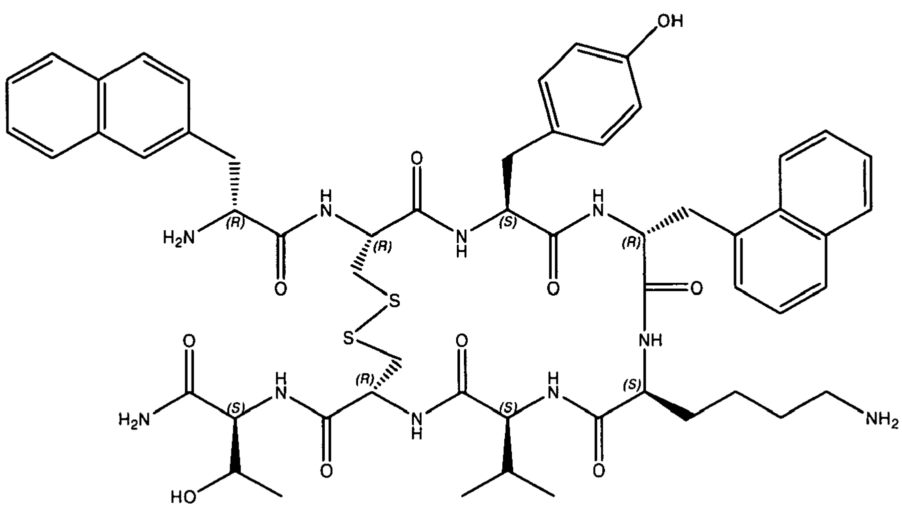

Le peptide ouvert est obtenu selon le protocole décrit précédemment par la succession de déprotection du groupement Fmoc (9-fluorènylméthyloxycarbonyl) et couplage de l'acide aminé suivant sur support solide dans l'ordre suivant : Fmoc-L-Thr(tBu)-OH puis Fmoc-L-Cys(Trt)-OH puis Fmoc-L-Val-OH puis Fmoc-L-Lys(Boc)-OH puis Fmoc-β-naphthyl)-D-Ala-OH puis Fmoc-L-Tyr(tBu)-OH puis Fmoc-L-Cys(Trt)-OH et enfin Boc-β-(2-naphthyl)-D-Ala-OH.

Le composé est cyclisé par la formation d'un pont disulfure, puis clivé de la résine, purifié, et enfin obtenu sous forme d'acétate selon le mode opératoire décrit précédemment pour conduire au composé H-D-2-$Nal^1$-cyclo ($Cys^2$-$Tyr^3$-D-$Val^4$-$Lys^5$-$Val^6$-$Cys^7$) $Thr^8$-$NH_2$•2 $CH_3COOH$.

HPLC : tr = 8,9 min

$^1$H NMR (400 Mhz, $D_2O$) : δ -0,10-0,04 (m, 1H); 0,22-0,34 (m, 1H); 0,87 (d, J = 4,6, 3H); 0,89 (d, J = 4,6, 3H); 0,98-1,10 (m, 1H); 1,18 (d, J = 6,4, 3H); 1,20-1,30 (m, 1H); 1,40-1,52 (m, 1H); 1,95 (s, 6H); 2,06-2,18 (m, 1H); 2,42 (dd, J = 14,9, J = 3,9, 1H); 2,57 (dd, J = 14,9, J = 8,8, 1H); 2,64 (t, J = 8,0, 2H); 2,72 (dd, J = 14,8, J = 4,8, 1H); 2,78 (dd, J = 10,8, J = 4,8, 1H); 2,89 (dd, J = 13,3, J = 8,3, 1H); 2,89 (dd, J = 13,6, J = 6,8, 1H); 3,26 (d, J = 8,4, 2H); 3,34 (dd, J = 13,4, J = 9,0, 1H); 3,48 (dd, J = 13,7, J = 6,0, 1H); 3,68 (dd, J = 10,8, J = 3,7, 1H); 3,90 (d, J = 9,7, 1H); 4,21 (ddd, J = 12,7, J = 6,4, J = 4,0, 1H); 4,29 (d, J = 3,8, 1H); 4,31 (t, J = 8,6, 1H); 4,42 (dd, J = 8,7, J = 5,8, 1H); 4,66 (dd, J = 8,5, J = 6,8, 1H); 4,82-4,87 (m, 1H); 6,86 (d, J = 8,6, 2H); 7,14 (d, J = 8,6, 2H); 7,28 (d, J = 7,3, 1H); 7,45 (t, J = 7,7, 1H); 7,46 (d, J = 9,7, 1H); 7,53 (dd, J = 6,4, J = 3,5, 1H); 7,54 (dd, J = 6,4, J = 3,5, 1H); 7,57-7,65 (M, 2H); 7,78 (s, 1H); 7,84-7,97 (M, 6H).

HRMS ($H_2O$) m/z : 1129,4578 [M+Na]+ (calc. 1129,4516) C56 H70 Na N10 O10 S2

***Exemple 7*** : H-D-2-$Nal^1$-cyclo($Cys^2$-$Tyr^3$-D-$Val^4$-$Lys^5$-$Val^6$-$Cys^7$)-$Thr^8$ $NH_2$•2 $CH_3COOH$

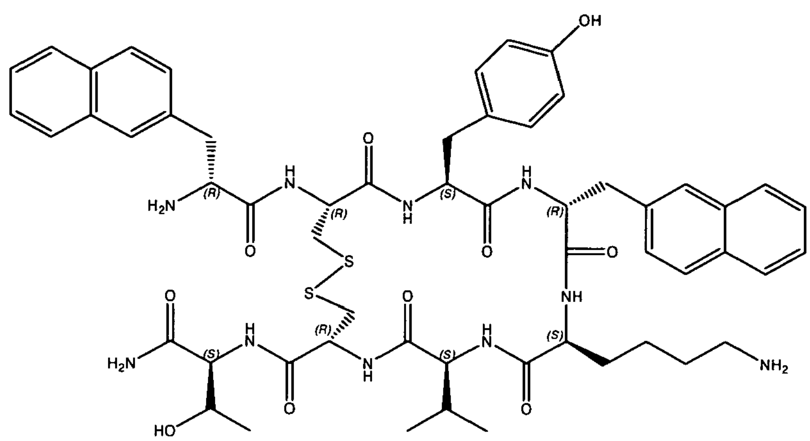

Le peptide ouvert est obtenu selon le protocole décrit précédemment par la succession de déprotection du groupement Fmoc (9-fluorènylméthyloxycarbonyl) et couplage de l'acide aminé suivant sur support solide dans l'ordre suivant : Fmoc-L-Thr(tBu)-OH puis Fmoc-L-Cys(Trt)-OH puis Fmoc-L-Val-OH puis Fmoc-L-Lys(Boc)-OH puis Fmoc-β-(2-naphthyl)-D-Ala-OH puis Fmoc-L-Tyr(tBu)-OH puis Fmoc-L-Cys(Trt)-OH et enfin Boc -β-(2-naphthyl)-D-Ala-OH.

Le composé est cyclisé par la formation d'un pont disulfure, puis clivé de la résine, purifié, et enfin obtenu sous forme d'acétate selon le mode opératoire décrit précédemment pour conduire au composé H-D-2-$Nal^1$-cyclo ($Cys^2$-$Tyr^3$-D-$Val^4$-$Lys^5$-$Val^6$-$Cys^7$) $Thr^8$-$NH_2$•2 $CH_3COOH$.

HPLC : tr = 14,5 min

HRMS ($H_2O$) m/z : 1129,4651 [M+H]+ C56 H69 N 10 O 10 S2

***Exemple 8*** : H-D-2-$Nal^1$-cyclo($Cys^2$-$Tyr^3$-β-(9-anthryl)-D-$Ala^4$-$Lys^5$-$Val^6$-$Cys^7$)-$Thr^8$-$NH_2$•2 $CH_3COOH$

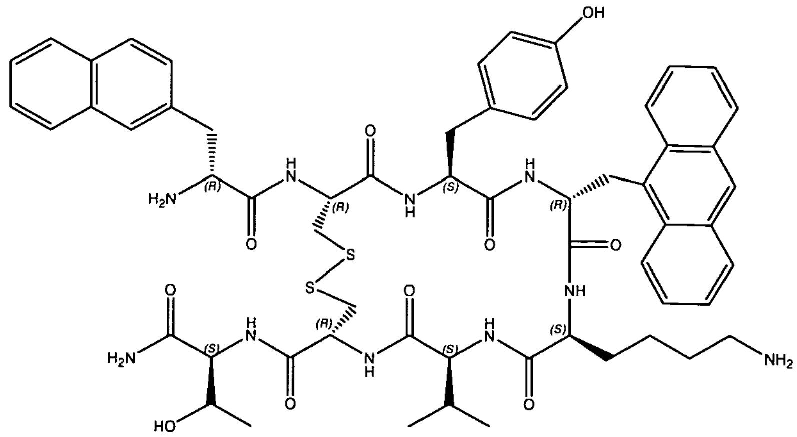

Le peptide ouvert est obtenu selon le protocole décrit précédemment par la succession de déprotection du groupement Fmoc (9-fluorènylméthyloxycarbonyl) et couplage de l'acide aminé suivant sur support solide dans l'ordre suivant : Fmoc-L-Thr(tBu)-OH puis Fmoc-L-Cys(Trt)-OH puis Fmoc-L-Val-OH puis Fmoc-L-Lys(Boc)-OH puis Fmoc-β-(9-anthryl)-D-Ala-OH puis Fmoc-L-Tyr(tBu)-OH puis Fmoc-L-Cys(Trt)-OH et enfin Boc-β-(2-naphthyl)-D-Ala-OH.

Le composé est cyclisé par la formation d'un pont disulfure, puis clivé de la résine, purifié, et enfin obtenu sous forme d'acétate selon le mode opératoire décrit précédemment pour conduire au composé H-D-2-$Nal^1$-cyclo($Cys^2$-$Tyr^3$-β-(9-anthryl)- D-$Ala^4$-$Lys^5$-$Val^6$-$Cys^7$)-$Thr^8$-$NH_2$•2 $CH_3COOH$.

HPLC : tr= 10,1 min

$^1$H NMR (400 Mhz, $D_2O$) : δ -0,68- -0,58 (m, 1H); -0,36- -0,24 (m, 1H); 0,68-0,72 (m, 1H); 0,83 (d, J = 4,6, 3H); 0,84 (d, J = 4,7, 0,92-1,04 (M, 2H); 1,06-1,12 (m, 1H); 1,18 (d, J = 6,4, 3H); 2,0 (s, 6H); 2,0-2,08 (m, 1H); 2,4 (dd, J = 14,7, J = 4,3, 1H); 2,44-2,54 (M, 3H); 2,78 (d, J = 7,8, 2H); 2,90 (dd, J = 13,2, J = 9,6, 1H); 3,01 (dd, J = 13,3, J = 6,2, 1H); 3,26 (dd, J = 10,4, J = 4,2, 1H); 3,30-3,36 (m, 1H); 3,38-3,52 (M, 2H); 3,84 (d, J = 9,3, 1H); 3,89 (d, J = 13,5, 1H); 4,20-4,30 (M, 2H); 4,32 (d, J = 3,8, 1H); 4,42 (dd, J = 9,1, J = 5,8, 1H); 4,66-4,76 (m, 2H); 6,97 (d, J = 8,6, 2H); 7,23 (d, J = 8,4, 2H); 7,44 (d, J = 8,6, 1H); 7,48-7,60 (M, 5H); 7,75 (s, 1H); 7,82-7,94 (M, 5H); 8,05 (d, J = 8,0, 2H); 8,4 (s, 1H).

HRMS ($H_2O$) m/z : 1157,4919 [M+H]+ (calc. 1157,4953) C60 H73 N10 010 S2

***Exemple 9 :*** H-D-2-$Nal^1$-cyclo($Cys^2$-$Tyr^3$-β-(2-fluorenyl)-D-$Ala^4$-$Lys^5$-$Val^6$-$Cys^7$)-$Thr^8$-$NH_2$•2 $CH_3COOH$

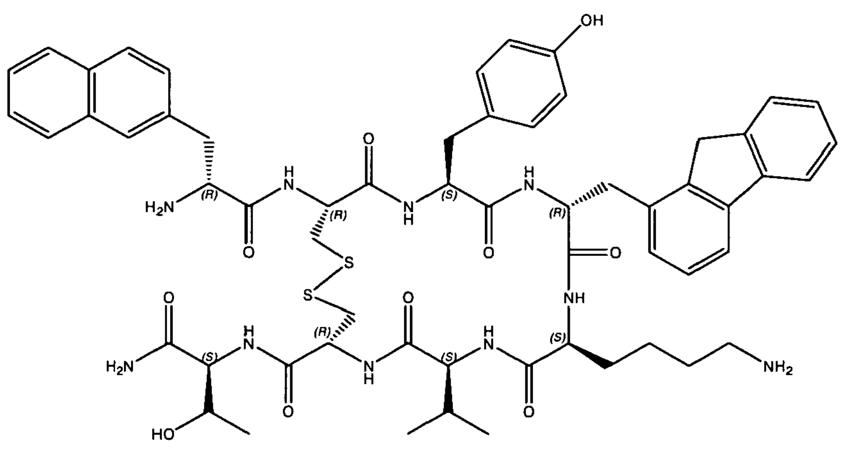

Le peptide ouvert est obtenu selon le protocole décrit précédemment par la succession de déprotection du groupement Fmoc (9-fluorènylméthyloxycarbonyl) et couplage de l'acide aminé suivant sur support solide dans l'ordre suivant : Fmoc-L-Thr(tBu)-OH puis Fmoc-L-Cys(Trt)-OH puis Fmoc-L-Val-OH puis Fmoc-L-Lys(Boc)-OH puis Fmoc-β-(2-fluorènyl)-D-Ala-OH puis Fmoc-L-Tyr(tBu)-OH puis Fmoc-L-Cys(Trt)-OH et enfin Boc-β-(2-naphthyl)-D-Ala-OH.

Le composé est cyclisé par la formation d'un pont disulfure, puis clivé de la résine, purifié, et enfin obtenu sous

forme d'acétate selon le mode opératoire décrit précédemment pour conduire au composé H-D-2-Nal[1]-cyclo(Cys[2]-Tyr[3]-β-(2-fluorènyl)-D-Ala[4]-Lys[5]-Val[6]-Cys[7])-Thr[8]-$NH_2$•2 $CH_3COOH$.

HPLC : tr = 9,8 min

$^1$H NMR (400 Mhz, $D_2O$) : δ 0,08-0,20 (m, 1H); 0,32-0,44 (m, 1H); 0,88 (d, J = 6,7, 3H); 0,91 (d, J = 6,6, 3H); 0,96 (t, J = 7,8, 2H); 1,17 (d, J = 6,4, 3H); 1,48-1,60 (m, 1H); 1,96 (s, 6H); 1,80-2,08 (M, 2H); 2,10-2,20 (m, 1H); 2,45 (dd, J = 14,7, J = 3,7, 1H); 2,61 (dd, J = 14,8, J = 9,7, 1H); 2,68 (dd, J = 14,8, J = 3,7, 1H); 2,76 (d, J = 11,0, 1H); 2,81 (dd, J = 11,9, J = 9,2, 1H); 2,88-3,00 (M, 3H); 3,35 (dd, J = 13,7, J = 8,8, 1H); 3,46 (dd, J = 13,7, J = 5,8, 1H); 3,80 (dd, J = 11,0, J = 3,2, 1H); 3,88 (s, 2H); 3,92 (d, J = 9,8, 1H); 4,19 (dd, J = 6,5, J = 3,7, 1H); 4,21 (dd, J = 11,8, J = 5,4, 1H); 4,29 (d, J = 3,8, 1H); 4,41 (dd, J = 8,8, J = 5,8, 1H); 4,63 (t, J = 7,5, 1H); 4,86 (dd, J = 11,7, J = 5,8, 1H); 4,90 (dd, J = 11,7, J = 5,8, 1H); 6,81 (d, J = 8,6, 2H); 7,11 (d, J = 8,6, 2H); 7,17 (d, J = 8,0, 1H); 7,43 (d, J =1,0, 1H); 7,7 (dd, J = 7,4, J = 1,1, 1H); 7,40-7,48 (M, 2H); 7,52 (dd, J = 9,9, J = 3,5, 1H); 7,52 (dd, J = 9,9, J = 3,1, 1H); 7,62 (d, J = 7,3, 1H); 7,78 (s, 1H); 7,79 (d, J = 7,8, 1H); 7,82-7,92 (M, 4H).

HRMS ($H_2O$) m/z : 1167,4774 [M+Na]+ (calc. 1167,4772) C59 H72 Na N10 O10 S2

***Exemple 10*** : H-D-2-Nal[1]-cyclo(Cys[2]-Tyr[3]-D-Phg[4]-Lys[5]-Val[6]-Cys[7])-Thr[8]-$NH_2$•2 $CH_3COOH$

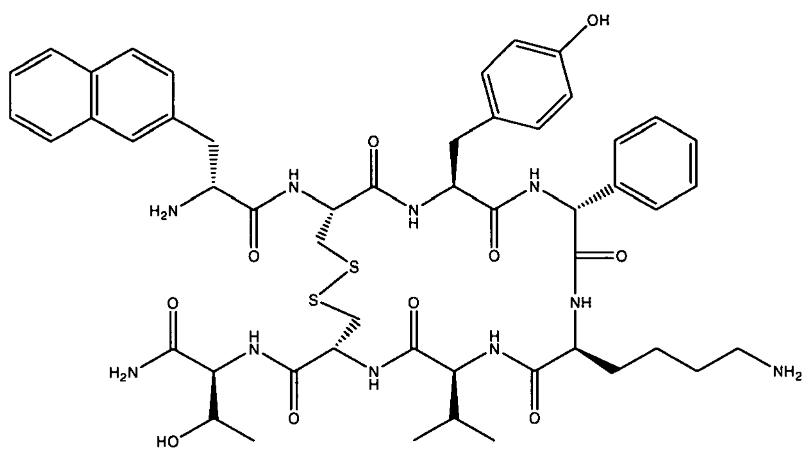

Le peptide ouvert est obtenu selon le protocole décrit précédemment par la succession de déprotection du groupement Fmoc (9-fluorènylméthyloxycarbonyl) et couplage de l'acide aminé suivant sur support solide dans l'ordre suivant : Fmoc-L-Thr(tBu)-OH puis Fmoc-L-Cys(Trt)-OH puis Fmoc-L-Val-OH puis Fmoc-L-Lys(Boc)-OH puis Fmoc-D-Phg-OH puis Fmoc-L-Tyr(tBu)-OH puis Fmoc-L-Cys(Trt)-OH et enfin Boc-β-(2-naphthyl)-D-Ala-OH.

Le composé est cyclisé par la formation d'un pont disulfure, puis clivé de la résine, purifié, et enfin obtenu sous forme d'acétate selon le mode opératoire décrit précédemment pour conduire au composé H-D-2-Nal[1]-cyclo (Cys[2]-Tyr[3]-D-Phg[4]-Lys[5]-Val[6]-Cys[7])-Thr[8]-$NH_2$•2 $CH_3COOH$.

HPLC : tr = 8,9 min

$^1$H NMR (400 Mhz, $D_aO$) : δ 0,93 (d, J = 7,0, 3H); 0,96 (d, J = 6,8, 3H); 1,10-1,20 (M, 2H); 1,20 (d, J = 6,4, 3H); 1,47 (q, J = 7,8, 2H); 1,61-1,72 (m, 1H); 1,82-1,92 (m, 1H); 1,97 (s, 6H); 2,12-2,20 (m, 1H); 2,40 (t, J = 8,3, 1H); 2,48 (d, J = 6,5, 2H); 2,68-2,89 (M, 6H); 2,95 (dd, J = 13,3, J = 6,0, 1H); 3,31 (dd, J = 13,0, J = 9,1, 1H); 3,42-3,51 (m, 1H); 4,11 (d, J = 8,4, 1H); 4,14 (dd, J = 10,0, J = 4,2, 1H); 4,39 (dd, J = 9,1, J = 6,0, 1H); 4,60 (dd, J = 9,2, J = 6,5, 1H); 4,66 (t, J = 6,6, 1H); 4,70-4,78 (M, 2H); 6,69 (d, J = 8,1, 2H); 7,03 (d, J = 8,4, 2H); 7,15 (d, J = 6,4, 2H); 7,32-7,42 (M, 3H); 7,46 (dd, J = 8,7, J = 0,5, 1H); 7,52-7,60 (M, 2H); 7,78 (ls, 1H); 7,88-7,98 (M, 3H).

HRMS ($H_2O$) m/z : 1065,4276 [M+Na]+ (calc. 1065,4303) C51 H66 Na N10 O10 S2

***Exemple 11*** **:** H-D-2-Nal[1]-cyclo(Cys[2]-Tyr[3]-D-Homophe[4]-Lys[5]-Val[6]-Cys[7])-Thr[8]-$NH_2$•2 $CH_3COOH$

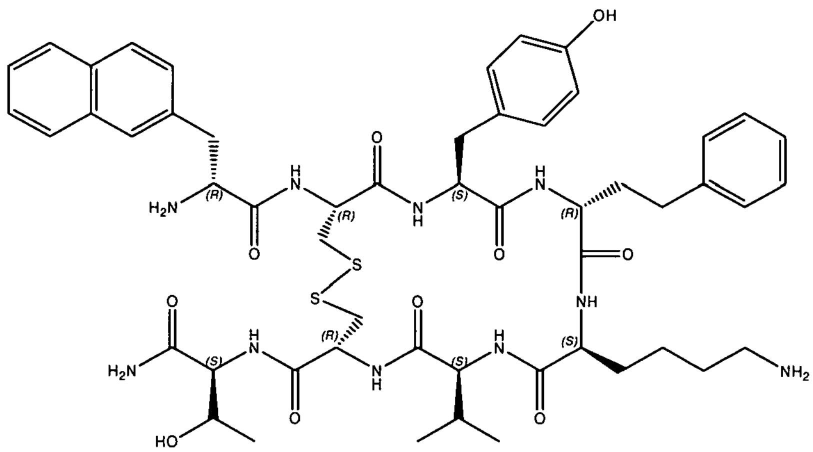

Le peptide ouvert est obtenu selon le protocole décrit précédemment par la succession de déprotection du groupement Fmoc (9-fluorènylméthyloxycarbonyl) et couplage de l'acide aminé suivant sur support solide dans l'ordre suivant : Fmoc-L-Thr(tBu)-OH puis Fmoc-L-Cys(Trt)-OH puis Fmoc-L-Val-OH puis Fmoc-L-Lys(Boc)-OH puis Fmoc-D-Homophe-OH puis Fmoc-L-Tyr(tBu)-OH puis Fmoc-L-Cys(Trt)-OH et enfin Boc-β-(2-naphthyl)-D-Ala-OH.

Le composé est cyclisé par la formation d'un pont disulfure, puis clivé de la résine, purifié, et enfin obtenu sous forme d'acétate selon le mode opératoire décrit précédemment pour conduire au composé H-D-2-Nal$^1$-cyclo (Cys$^2$-Tyr$^3$-D-Val$^4$-Lys$^5$-Val$^6$-Cys$^7$)-Thr$^8$-$NH_2$•2 $CH_3COOH$.

HPLC : tr = 8,87 min

$^1$H NMR (400 Mhz, $D_2O$) : δ 0,89 (d, J = 6,7, 3H); 0,92 (d, J = 6,7, 3H); 1,17 (d, J = 6,4, 3H); 1,26-1,36 (M, 2H); 1,54-1,68 (M, 3H); 1,76-1,84 (M, 2H); 1,86-1,92 (m, 1H); 2,08-2,18 (m, 1H); 1,94 (s, 6H); 2,27 (ddd, J = 14,0, J = 7,9, J = 5,9, 1H); 2,36 (ddd, J = 14,2, J = 6,4, J = 5,6, 1H); 2,48 (dd, J = 14,7, J = 4,8, 1H); 2,56 (dd, J = 14,8, J = 8,8, 1H); 2,72 (d, J = 3,7, 1H); 2,74 (s, 1H); 2,84-2,82 (M, 3H); 2,96 (dd, J = 13,4, J = 5,9, 1H); 3,34 (dd, J = 13,8, J = 8,9, 1H); 3,47 (J = 13,7, J = 5,8, 1H); 3,88 (dd, J = 8,5, J = 6,3, 1H); 4,02 (d, J = 9,5, 1H); 4,14 (dd, J = 9,9, J = 4,3, 1H); 4,21 (dd, J = 6,5, J = 3,9, 1H); 4,30 (d, J = 3,9, 1H); 4,40 ( dd, J = 8,9, J = 6,0, 1H); 4,61 (dd, J = 9,9, J = 6,1, 1H); 4,80-4,88 (M, 2H); 6,82 (d, J = 8,6, 2H); 7,03 (dd, J = 6,9, J = 1,4, 1H); 7,14 (d, J = 8,6, 2H); 7,22-7,28 (m, 1H); 7,28-7,34 (M, 2H); 7,45 (dd, J = 8,5, J = 1,7, 1H); 7,52 (ddd, J = 6,9, J = 5,1, J = 2,0, 1H); 7,53 (ddd, J = 6,9, J = 5,1, J = 2,0, 1H), 7,78 (s, 1H); 7,86-7,93 (M, 3H).

HRMS ($H_2O$) m/z : 1129,4609 [M+H]+ (calc. 1093,4616) C53 H70 Na N10 O10 S2

***Exemple 12:*** H-D-2-Nal$^1$-cyclo(Cys$^2$-Tyr$^3$-*p*-Br-D-Phe$^4$-Lys$^5$-Val$^6$-Cys$^7$)-Thr$^8$-$NH_2$•2 $CH_3COOH$

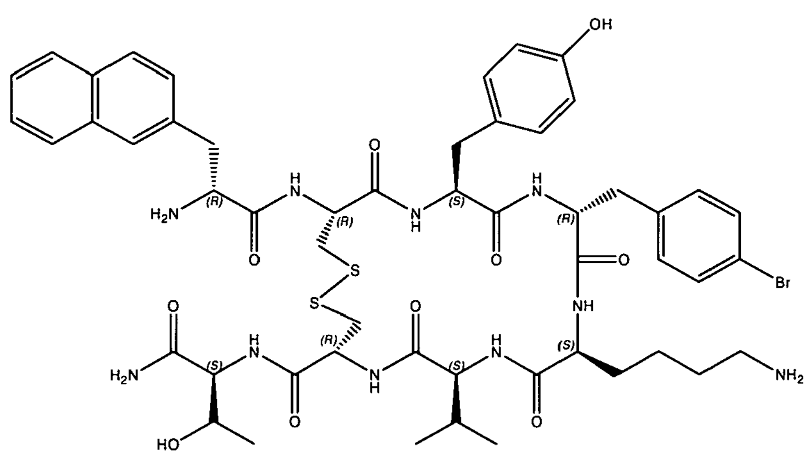

Le peptide ouvert est obtenu selon le protocole décrit précédemment par la succession de déprotection du groupement Fmoc (9-fluorènylméthyloxycarbonyl) et couplage de l'acide aminé suivant sur support solide dans l'ordre suivant : Fmoc-L-Thr(tBu)-OH puis Fmoc-L-Cys(Trt)-OH puis Fmoc-L-Val-OH puis Fmoc-L-Lys(Boc)-OH puis Fmoc-*p*-Br-D-Phe-OH puis Fmoc-L-Tyr(tBu)-OH puis Fmoc-L-Cys(Trt)-OH et enfin Boc-β-(2-naphthyl)-D-Ala-OH.

Le composé est cyclisé par la formation d'un pont disulfure, puis clivé de la résine, purifié, et enfin obtenu sous forme d'acétate selon le mode opératoire décrit précédemment pour conduire au composé H-D-2-$Nal^1$-cyclo ($Cys^2$-$Tyr^3$-D-$Val^4$-$Lys^5$-$Val^6$-$Cys^7$)-$Thr^8$ $NH_2$•2 $CH_3COOH$.

HPLC : tr = 9,5 min

$^1$H NMR (400 Mhz, $D_2O$) : δ 0,50-0,62 (m, 1H); 0,70-0,82 (m, 1H); 0,91 (d, J = 6,8, 3H); 0,99 (d, J = 6,6, 3H); 1,18 (d, J = 6,6, 3H); 1,30-1,42 (m, 1H); 1,42-1,52 (m, 2H); 1,70-1,80 (m, 1H) ; 1,96 (s, 6H); 2,12-2,28 (m, 1H); 2,47 (dd, J = 14,8, J = 3,6, 1H); 2,63 (dd, J = 14,6, J = 9,8, 1H); 2,68 (dd, J = 14,7, J = 3,7, 1H); 2,80-2,82 (M, 2H); 2,84-2,96 (M, 4H); 3,36 (dd, J = 13,7, J = 8,7, 1H); 3,48 (dd, J = 13,5, J = 6,2, 1H); 3,95 (dd, J = 11,1, J = 3,5, 1H); 3,98 (d, J = 10,0, 1H); 4,18-4,24 (M, 2H); 4,30 (d, J = 3,8, 1H); 4,42 (dd, J = 8,6, J = 6,0, 1H); 4,62 (t, J = 7,1, 1H); 4,88-4,94 (M, 2H); 6,82 (d, J = 8,6, 2H); 7,07 (d, J = 8,4, 2H); 7,10 (d, J = 8,6, 2H); 7,48-7,52 (M, 3H); 7,53 (dd, J = 9,5, J = 3,3, 1H); 7,55 (dd, J = 9,5, J = 3,7, 1H); 7,80 (s, 1H); 7,84-7,94 (M, 3H).

HRMS ($H_2O$) m/z : 1157,3549 et 1159,3585 [M+Na]+ (calc. 1157,3564 et 1159,3544) C52 H67 Br Na N10 010 S2

***Exemple 13:*** H-D-2-$Nal^1$-cyclo($Cys^2$-$Tyr^3$-*p*-F-D-$Phe^4$-$Lys^5$-$Val^6$-$Cys^7$)-$Thr^8$-$NH_2$•2 $CH_3COOH$

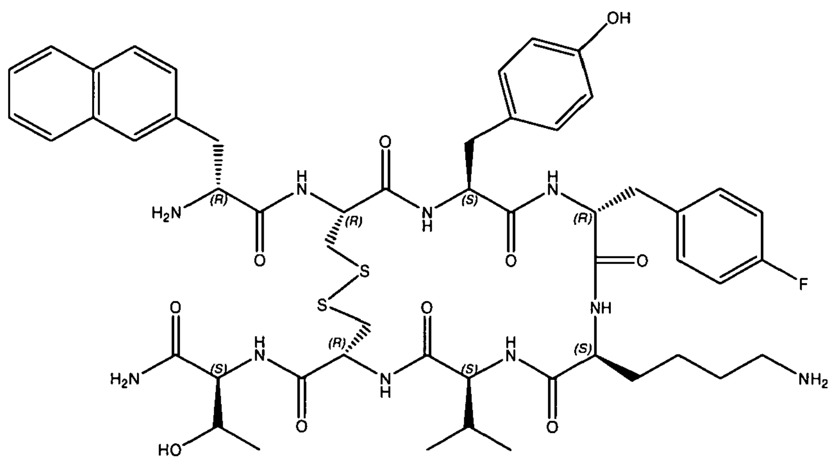

Le peptide ouvert est obtenu selon le protocole décrit précédemment par la succession de déprotection du groupement Fmoc (9-fluorènylméthyloxycarbonyl) et couplage de l'acide aminé suivant sur support solide dans l'ordre suivant : Fmoc-L-Thr(tBu)-OH puis Fmoc-L-Cys(Trt)-OH puis Fmoc-L-Val-OH puis Fmoc-L-Lys(Boc)-OH puis Fmoc-*p*-F-D-Phe-OH puis Fmoc-L-Tyr(tBu)-OH puis Fmoc-L-Cys(Trt)-OH et enfin Boc-β-(2-naphthyl)-D-Ala-OH.

Le composé est cyclisé par la formation d'un pont disulfure, puis clivé de la résine, purifié, et enfin obtenu sous forme d'acétate selon le mode opératoire décrit précédemment pour conduire au composé H-D-2-$Nal^1$-cyclo ($Cys^2$-$Tyr^3$-D-$Val^4$-$Lys^5$-$Val^6$-$Cys^7$)-$Thr^8$-$NH_2$•2 $CH_3COOH$.

HPLC : tr = 8,7 min

$^1$H NMR (400 Mhz, $D_2O$) : δ 0,50-0,62 (m, 1H); 0,72-0,84 (m, 1H); 0,91 (d, J = 6,8, 3H); 0,93 (d, J = 6,6, 3H); 1,18 (d, J = 6,4, 3H); 1,30-1,50 (M, 4H); 1,70-1,80 (m, 1H); 2,02 (s, 6H); 2,14-2,26 (m, 1H); 2,47 (dd, J = 15,0, J = 3,8, 1H); 2,63 (dd, J = 14,8, J = 9,9, 1H); 2,68 (dd, J = 14,8, J = 3,4, 1H); 2,74-2,98 (M, 6H); 3,37 (dd, J = 13,8, J = 8,8, 1H); 3,48 (dd, J = 13,7, J = 6,0, 1H); 3,92-4,02 (M, 2H); 4,18-4,24 (M, 2H); 4,30 (d, J = 3,8, 1H); 4,43 (dd, J = 8,7, J = 5,9, 1H); 4,62 (t, J = 7,5, 1H); 4,88-4,96 (M, 2H); 6,81 (d, J = 8,4, 2H); 7,04-7,18 (M, 6H); 7,47 (dd, J = 8,5, J = 1,5, 1H); 7,54 (dd, J = 9,7, J = 3,4, 1H); 7,54 (dd, J = 9,6, J = 3,4, 1H); 7,80 (s, 1H); 7,84-7,96 (M, 3H).

HRMS ($H_2O$) m/z : 1097,4332 [M+Na]+ (calc. 1097,4365) C52 H67 F N10 O10 S2

***Exemple 14 :*** H-D-2-$Nal^1$-cyclo($Cys^2$-$Tyr^3$-D-$Tyr^4$-$Lys^5$-$Val^6$-$Cys^7$)-$Thr^8$-$NH_2$•2 $CH_3COOH$

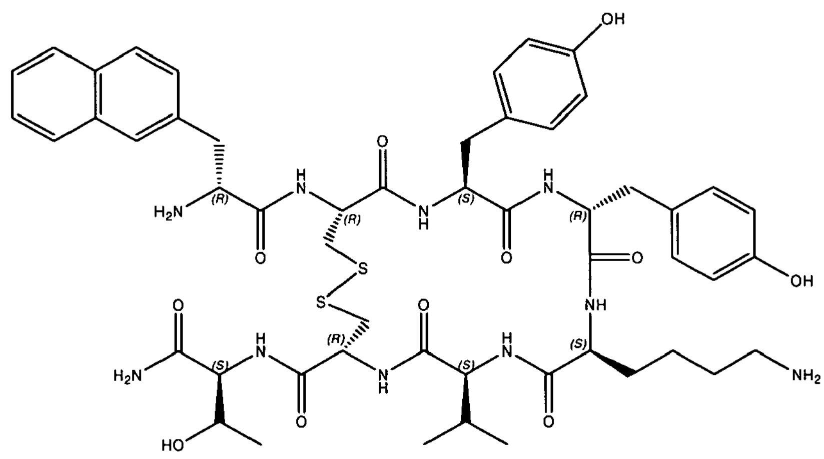

Le peptide ouvert est obtenu selon le protocole décrit précédemment par la succession de déprotection du groupement Fmoc (9-fluorènylméthyloxycarbonyl) et couplage de l'acide aminé suivant sur support solide dans l'ordre suivant : Fmoc-L-Thr(tBu)-OH puis Fmoc-L-Cys(Trt)-OH puis Fmoc-L-Val-OH puis Fmoc-L-Lys(Boc)-OH puis Fmoc-D-Tyr-OH puis Fmoc-L-Tyr(tBu)-OH puis Fmoc-L-Cys(Trt)-OH et enfin Boc-β-(2-naphthyl)-D-Ala-OH.

Le composé est cyclisé par la formation d'un pont disulfure, puis clivé de la résine, purifié, et enfin obtenu sous forme d'acétate selon le mode opératoire décrit précédemment pour conduire au composé H-D-2-$Nal^1$-cyclo($Cys^2$-$Tyr^3$-D-$Val^4$-$Lys^5$-$Val^6$-$Cys^7$)-$Thr^8$-$NH_2$•2 $CH_3COOH$.

HPLC : tr = 7,7 min

$^1$H NMR (400 Mhz, $D_2O$) : δ 0,50-0,64 (m, 1H); 0,66-0,80 (m, 1H); 0,91 (d, J = 6,6, 3H); 0,93 (d, J = 6,6, 3H); 1,20 (d, J = 6,4, 3H); 1,28-1,48 (M, 3H); 1,66-1,78 (m, 1H); 1,89 (s, 6H); 2,12-2,24 (m, 1H); 2,41 (dd, J = 14,8, J = 3,7, 1H); 2,55 (dd, J = 14,8, J = 9,5, 1H); 2,64-2,96 (M, 7H); 3,20 (dd, J = 13,4, J = 9,1, 1H); 3,33 (dd, J = 13,2, J = 5,7, 1H); 3,92 (dd, J = 11,0, J = 3,2, 1H); 3,97 (d, J = 9,7, 1H); 4,10-4,20 (M, 2H); 4,24 (qd, J = 10,5, J = 6,4, 1H); 4,32 (d, J = 3,8, 1H); 4,60 (t, J = 7,3, 1H); 6,80 (t, J = 8,3, 4H); 7,02 (d, J = 8,2, 2H); 7,09 (d, J = 8,4, 2H); 7,43 (d, J = 8,4, 1H); 7,48-7,56 (M, 2H); 7,73 (s, 1H); 7,80-7,92 (M, 4H).

HRMS ($H_2O$) m/z : 1073,4578 [M+H]+ (calc. 1073,4589) C52 H69 N10 011 S2

***Exemple 15:*** H-D-2-$Nal^1$-cyclo($Cys^2$-$Tyr^3$-*m*-F-D-$Phe^4$-$Lys^5$-$Val^6$-$Cys^7$)-$Thr^8$-$NH_2$•2 $CH_3COOH$

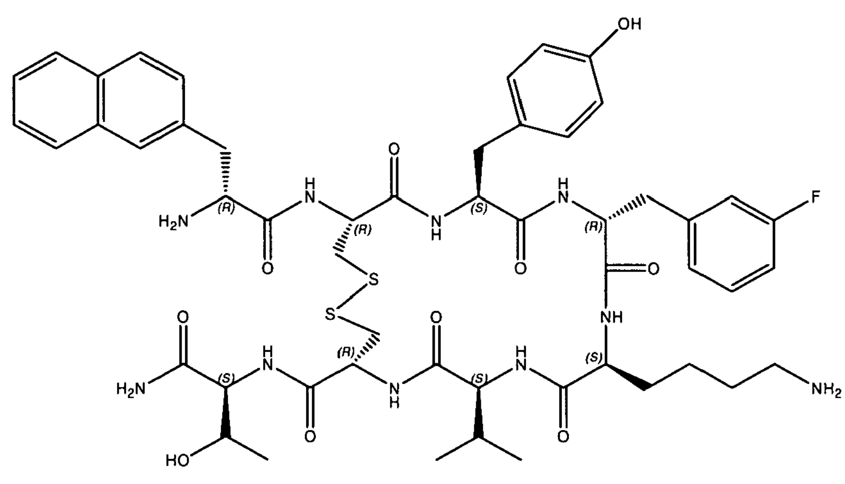

Le peptide ouvert est obtenu selon le protocole décrit précédemment par la succession de déprotection du groupement Fmoc (9-fluorènylméthyloxycarbonyl) et couplage de l'acide aminé suivant sur support solide dans l'ordre suivant : Fmoc-L-Thr(tBu)-OH puis Fmoc-L-Cys(Trt)-OH puis Fmoc-L-Val-OH puis Fmoc-L-Lys(Boc)-OH puis Fmoc-m-F-D-Phe-OH puis Fmoc-L-Tyr(tBu)-OH puis Fmoc-L-Cys(Trt)-OH et enfin Boc-β-(2-naphthyl)-D-Ala-OH.

Le composé est cyclisé par la formation d'un pont disulfure, puis clivé de la résine, purifié, et enfin obtenu sous forme d'acétate selon le mode opératoire décrit précédemment pour conduire au composé HH-D-2-Nal$^1$-cyclo (Cys$^2$-Tyr$^3$-D-Val$^4$-Lys$^5$-Val$^6$-Cys$^7$)-Thr$^8$-$NH_2$•2 $CH_3COOH$.

HPLC : tr = 8,1 min

$^1$H NMR (400 Mhz, $D_2O$) : δ 0,60-0,72 (m, 1H); 0,74-0,88 (m, 1H); 0,92 (d, J = 6,7, 3H); 0,95 (d, J = 6,4, 3H); 1,20 (d, J = 6,7, 3H); 1,34-1,51 (M, 3H); 1,72-1,82 (m, 1H); 1,90 (s, 1H); 2,15-2,24 (m, 1H); 2,47 (dd, J = 14,7, J = 3,9, 1H); 2,61 (dd, J = 14,8, J = 9,8, 1H); 2,70 (dd, J = 14,5, J = 3,6, 1H); 2,74-2,84 (M, 4H); 2,86-2,94 (M, 3H); 3,32 (dd, J = 13,8, J = 8,8, 1H); 3,44 (dd, J = 13,7, J = 5,7, 1H); 4,00 (d, J = 9,9, 1H); 4,01 (d, J = 10,8, 1H); 4,23 (dd, J = 6,4, J = 3,8, 1H); 4,25 (dd, J = 10,4, J = 6,4, 1H); 4,32-4,36 (M, 2H); 4,61 (dd, J = 7,1, J = 7,9, 1H); 4,88 (dd, J = 10,0, J = 3,9, 1H); 4,91 (dd, J = 11,5, J = 3,5, 1H); 6,82 (d, J = 8,5, 2H); 6,93 (Id, J = 9,7, 1H); 6,98 (d, J = 7,7, 1H); 7,01-7,08 (m, 1H); 7,10 (d, J = 8,5, 2H); 7,35 (dd, J = 14,7, J = 7,9, 1H); 7,47 (dd, J = 8,6, J = 1,3, 1H); 7,52-7,57 (M, 2H); 7,80 (Is, 1H); 7,86-7,89 (m, 1H); 7,90-7,94 (M, 2H).
HRMS ($H_2O$ + ACN) m/z : 1075,4492 [M+H]+ (calc, 1075,4545) C52 H68 F N10 O10 S2

***Exemple 16:*** H-D-2-Nal$^1$-cyclo(Cys$^2$-Tyr$^3$-*o*-Val$^4$-Lys$^5$-Val$^6$-Cys$^7$)-Thr$^8$-$NH_2$•2 $CH_3COOH$

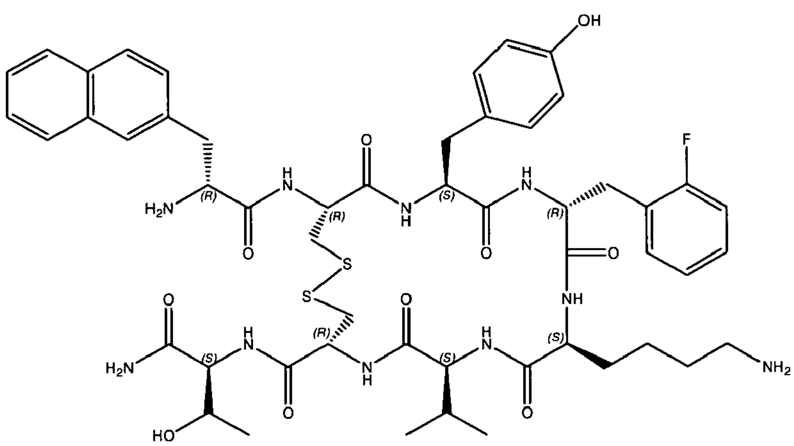

Le peptide ouvert est obtenu selon le protocole décrit précédemment par la succession de déprotection du groupement Fmoc (9-fluorènylméthyloxycarbonyl) et couplage de l'acide aminé suivant sur support solide dans l'ordre suivant : Fmoc-L-Thr(tBu)-OH puis Fmoc-L-Cys(Trt)-OH puis Fmoc-L-Val-OH puis Fmoc-L-Lys(Boc)-OH puis Fmoc-o-F-D-Phe-OH puis Fmoc-L-Tyr(tBu)-OH puis Fmoc-L-Cys(Trt)-OH et enfin Boc-β-(2-naphthyl)-D-Ala-OH.

Le composé est cyclisé par la formation d'un pont disulfure, puis clivé de la résine, purifié, et enfin obtenu sous forme d'acétate selon le mode opératoire décrit précédemment pour conduire au composé H-D-2-Nal$^1$-cyclo (Cys$^2$-Tyr$^3$-*o*-F-D-Phe$^4$-Lys$^5$-Val$^6$-Cys$^7$)-Thr$^8$-$NH_2$•2 $CH_3COOH$.

HPLC : tr = 8,1 min

$^1$H NMR (400 Mhz, $D_2O$) : δ 0,58-0,70 (m, 1H); 0,74-0,86 (m, 1H); 0,91 (d, J = 6,6, 3H); 0,94 (d, J = 6,7, 3H); 1,19 (d, J = 6,4, 3H); 1,33-1,51 (M, 4H); 1,70-1,80 (m, 1H); 1,97 (s, 6H); 2,12-2,25 (m, 1H); 2,47 (Id, J = 13,3, 1H); 2,61 (dd, J = 14,6 , J = 9,3, 1H); 2,64-2,86 (M, 4H); 2,86-2,96 (M, 3H); 3,31-3,41 (m, 1H); 3,42-3,52 (m, 1H); 3,99 (d, J = 10,0, 2H); 4,18-4,22 (m, 1H); 4,28 (dd, J = 10,3 , J = 6,1, 1H); 4,31 (d, J = 4,3, 1H); 4,42 (Is, 1H); 4,62 (dd, J = 7,5 , J = 7,0, 1H); 4,83-4,93 (M, 2H); 6,82 (d, J = 7,9, 2H); 7,06-7,18 (M, 5H); 7,28-7,36 (m, 1H); 7,48 (Id, J = 7,3, 1H); 7,56 (Is, 2H); 7,80 (Is, 1H); 7,84-7,98 (M, 3H).
HRMS ($H_2O$) m/z : 1075,4514 [M+H]+ (calc, 1075,4545) C52 H68 N10 O10 S2

***Exemple 17:*** H-D-2-Nal$^1$-cyclo(Cys$^2$-Tyr$^3$-*di*F-D-Phe$^4$-Lys$^5$-Val$^6$-Cys$^7$)-Thr$^8$-$NH_2$•2 $CH_3COOH$

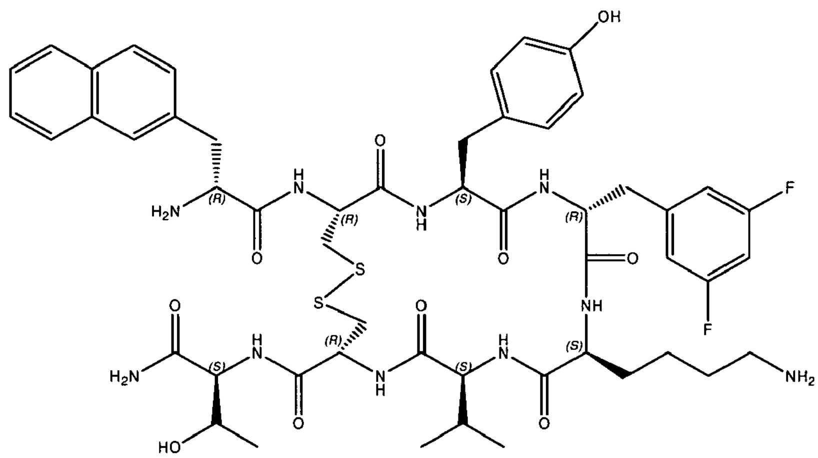

Le peptide ouvert est obtenu selon le protocole décrit précédemment par la succession de déprotection du groupement Fmoc (9-fluorènylméthyloxycarbonyl) et couplage de l'acide aminé suivant sur support solide dans l'ordre suivant : Fmoc-L-Thr(tBu)-OH puis Fmoc-L-Cys(Trt)-OH puis Fmoc-L-Val-OH puis Fmoc-L-Lys(Boc)-OH puis Fmoc-3,5-diF-D-Phe-OH puis Fmoc-L-Tyr(tBu)-OH puis Fmoc-L-Cys(Trt)-OH et enfin Boc-β-(2-naphthyl)-D-Ala-OH.

Le composé est cyclisé par la formation d'un pont disulfure, puis clivé de la résine, purifié, et enfin obtenu sous forme d'acétate selon le mode opératoire décrit précédemment pour conduire au composé H-D-2-Nal$^1$-cyclo (Cys$^2$-Tyr$^3$-*o*-F-D-Phe$^4$-Lys$^5$-Val$^6$-Cys$^7$)-Thr$^8$-$NH_2$•2 $CH_3COOH$.

HPLC : tr = 8,8 min

$^1$H NMR (400 Mhz, $D_2O$) : δ 0,75-0,85 (m, 1H); 0,92 (d, J = 6,6, 3H); 0,96 (d, J = 6,6, 3H); 1,20 (d, J = 6,6, 3H); 1,40-1,55 (M, 3H); 1,77-1,87 (m, 1H); 1,90 (s, 6H); 2,20 (dd, J = 13,2, J = 6,6, 1H); 2,49 (dd, J = 14,8, J = 4,0, 1H); 2,62 (dd, J = 14,7, J = 9,8, 1H); 2,70 (dd, J = 14,7, J = 3,8, 1H); 2,79 (dd, J = 14,7, J = 11,4, 1H); 2,81-2,95 (M, 6H); 3,34 (dd, J = 13,6, J = 9,0, 1H); 3,45 (dd, J = 13,6, J = 5,8, 1H); 4,01 (d, J = 9,9, 1H); 4,06 (dd, J = 10,8, J = 3,6, 1H); 4,20-4,28 (M, 2H); 4,33 (d, J = 3,8, 1H); 4,35 (dd, J = 8,8, J = 6,0, 1H); 4,61 (d, J = 7,7, 1H); 4,89 (dd, J = 9,8, J = 4,1, 1H); 4,92 (dd, J = 11,3, J = 3,6, 1H); 6,75-6,84 (M, 4H); 6,86-6,92 (m, 1H); 7,09 (d, J = 8,6, 2H); 7,48 (dd, J = 8,2, J = 1,1, 1H); 7,52-7,59 (M, 2H); 7,80 (s, 1H); 7,85-7,95 (M, 3H).

HRMS ($H_2O$) m/z : 1093,4406 [M+H]+ (calc. 1093,4451) C52 H67 N10 O10 F2 S2

***Exemple 18:*** H-D-2-Nal$^1$-cyclo(Cys$^2$-Tyr$^3$-*m*-Br-D-Phe$^4$-Lys$^5$-Val$^6$-Cys$^7$)-Thr$^8$-$NH_2$•2 $CH_3COOH$

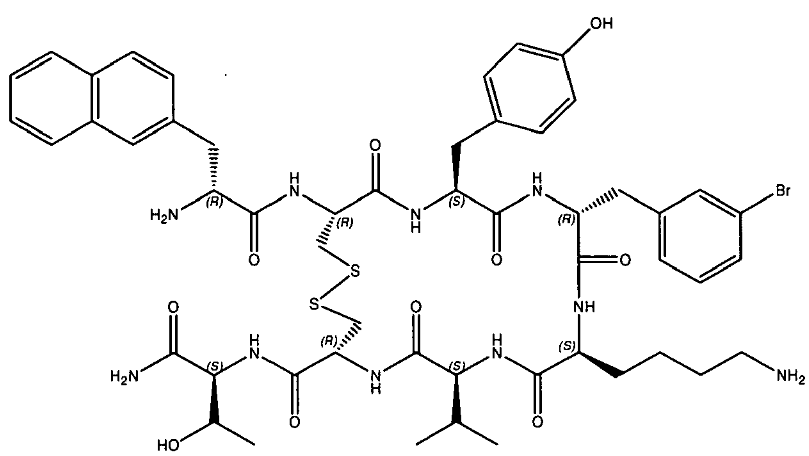

Le peptide ouvert est obtenu selon le protocole décrit précédemment par la succession de déprotection du groupement Fmoc (9-fluorènylméthyloxycarbonyl) et couplage de l'acide aminé suivant sur support solide dans l'ordre suivant : Fmoc-L-Thr(tBu)-OH puis Fmoc-L-Cys(Trt)-OH puis Fmoc-L-Val-OH puis Fmoc-L-Lys(Boc)-OH puis Fmoc-m-Br-D-Phe-OH puis Fmoc-L-Tyr(tBu)-OH puis Fmoc-L-Cys(Trt)-OH et enfin Boc-β-(2-naphthyl)-D-Ala-OH.

Le composé est cyclisé par la formation d'un pont disulfure, puis clivé de la résine, purifié, et enfin obtenu sous forme d'acétate selon le mode opératoire décrit précédemment pour conduire au composé H-D-2-$Nal^1$-cyclo ($Cys^2$-$Tyr^3$-*o*-F-D-$Phe^4$-$Lys^5$-$Val^6$-$Cys^7$)-$Thr^8$-$NH_2$•2 $CH_3COOH$.

HPLC : tr = 9,0 min

$^1$H NMR (400 Mhz, $D_2O$) : δ 0,52-0,68 (m, 1H); 0,76-0,90 (m, 1H); 0,92 (d, J = 6,8, 3H); 0,95 (d, J = 6,6, 3H); 1,21 (d, J = 6,4, 3H); 1,30-1,54 (M, 3H); 1,70-1,82 (m, 1H); 1,93 (s, 6H); 2,12-2,27 (m, 1H); 2,48 (dd, J = 14,8, J = 3,7, 1H); 2,63 (dd, J = 14,8, J = 9,7, 1H); 2,70 (dd, J = 14,5, J = 3,5, 1H); 2,74-2,99 (M, 7H); 2,28 (dd, J = 14,0, J = 9,1, 1H); 3,49 (dd, J = 13,7, J = 5,7, 1H); 3,95-4,05 (M, 2H); 4,19-4,27 (M, 2H); 4,32 (d, J = 3,8, 1H); 4,43 (dd, J = 8,7, J = 5,7, 1H); 4,63 (dd, J = 7,7, J = 7,2, 1H); 4,87-4,95 (m, 1H); 6,83 (d, J = 8,1, 2H); 7,11 (d, J = 8,4, 2H); 7,16 (d, J = 7,5, 1H); 7,27 (t, J = 7,8, 1H); 7,36 (s, 1H); 7,48 (s, 1H); 7,50 (s, 1H); 7,53-7,60 (M, 2H); 7,81 (s, 1H); 7,86-7,96 (M, 3H).

HRMS ($H_2O$) m/z : 1135,3741 et 1137,3704 [M+H]+ (calc. 1135,3745 and 1137,3724) C52 H68 Br N10 O10 S2

***Exemple 19 :*** H-D-2-$Nal^1$-cyclo($Cys^2$-$Tyr^3$-*o*-F-D-$Phe^4$-$Lys^5$-$Val^6$-$Cys^7$)-$Thr^8$-$NH_2$•2 $CH_3COOH$

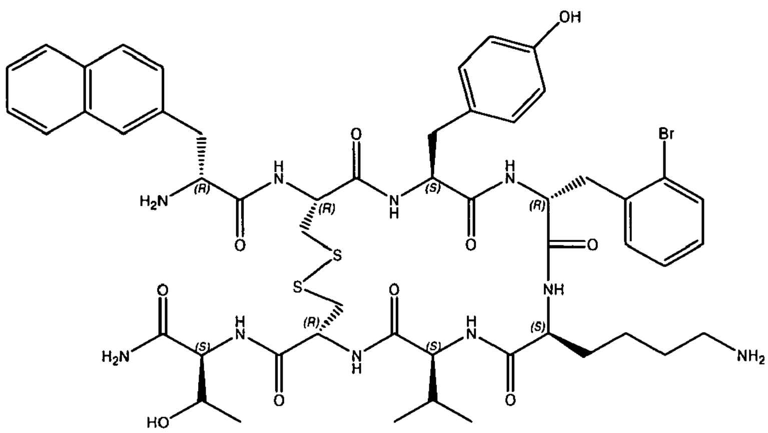

Le peptide ouvert est obtenu selon le protocole décrit précédemment par la succession de déprotection du groupement Fmoc (9-fluorènylméthyloxycarbonyl) et couplage de l'acide aminé suivant sur support solide dans l'ordre suivant : Fmoc-L-Thr(tBu)-OH puis Fmoc-L-Cys(Trt)-OH puis Fmoc-L-Val-OH puis Fmoc-L-Lys(Boc)-OH puis Fmoc-o-Br-D-Phe-OH puis Fmoc-L-Tyr(tBu)-OH puis Fmoc-L-Cys(Trt)-OH et enfin Boc-β-(2-naphthyl)-D-Ala-OH.

Le composé est cyclisé par la formation d'un pont disulfure, puis clivé de la résine, purifié, et enfin obtenu sous forme d'acétate selon le mode opératoire décrit précédemment pour conduire au composé H-D-2-$Nal^1$-cyclo ($Cys^2$-$Tyr^3$-*o*-Br-D-$Phe^4$-$Lys^5$-$Val^6$-$Cys^7$)-$Thr^8$-$NH_2$•2 $CH_3COOH$.

HPLC : tr = 9,3 min

$^1$H NMR (400 Mhz, $D_2O$) : δ 0,52-0,68 (m, 1H); 0,76-0,90 (m, 1H); 0,92 (d, J = 6,8, 3H); 0,95 (d, J = 6,6, 3H); 1,21 (d, J = 6,4, 3H); 1,30-1,54 (M, 3H); 1,70-1,82 (m, 1H); 1,93 (s, 6H); 2,12-2,27 (m, 1H); 2,48 (dd, J = 14,8, J = 3,7, 1H); 2,63 (dd, J = 14,8, J = 9,7, 1H); 2,70 (dd, J = 14,5, J = 3,5, 1H); 2,74-2,99 (M, 7H); 2,28 (dd, J = 14,0, J = 9,1, 1H); 3,49 (dd, J = 13,7, J = 5,7, 1H); 3,95-4,05 (M, 2H); 4,19-4,27 (M, 2H); 4,32 (d, J = 3,8, 1H); 4,43 (dd, J = 8,7, J = 5,7, 1H); 4,63 (dd, J = 7,7, J = 7,2, 1H); 4,87-4,95 (m, 1H); 6,83 (d, J = 8,1, 2H); 7,11 (d, J = 8,4, 2H); 7,16 (d, J = 7,5, 1H); 7,27 (t, J = 7,8, 1H); 7,36 (s, 1H); 7,48 (s, 1H); 7,50 (s, 1H); 7,53-7,60 (M, 2H); 7,81 (s, 1H); 7,86-7,96 (M, 3H).

HRMS ($H_2O$) m/z : 1135,3749 et 1137,3723 [M+H]+ (calc. 1135,3745 et 1137,3724) C52 H68 Br N10 O10 S2

***Exemple 20 :*** H-D-2-$Nal^1$-cyclo($Cys^2$-$Tyr^3$-*p*-nitro-D-$Phe^4$-$Lys^5$-$Val^6$-$Cys^7$)-$Thr^8$-$NH_2$•2 $CH_3COOH$

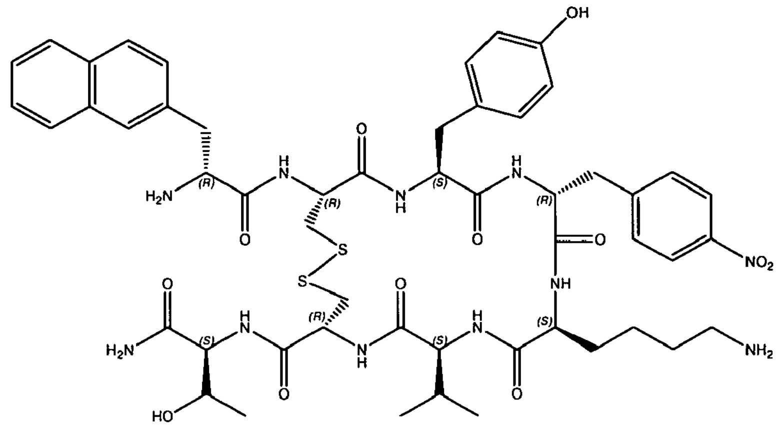

Le peptide ouvert est obtenu selon le protocole décrit précédemment par la succession de déprotection du groupement Fmoc (9-fluorènylméthyloxycarbonyl) et couplage de l'acide aminé suivant sur support solide dans l'ordre suivant : Fmoc-L-Thr(tBu)-OH puis Fmoc-L-Cys(Trt)-OH puis Fmoc-L-Val-OH puis Fmoc-L-Lys(Boc)-OH puis Fmoc-*p*-nitro-D-Phe-OH puis Fmoc-L-Tyr(tBu)-OH puis Fmoc-L-Cys(Trt)-OH et enfin Boc-β-(2-naphthyl)-D-Ala-OH.

Le composé est cyclisé par la formation d'un pont disulfure, puis clivé de la résine, purifié, et enfin obtenu sous forme d'acétate selon le mode opératoire décrit précédemment pour conduire au composé H-D-2-$Nal^1$-cyclo ($Cys^2$-$Tyr^3$-*p*-nitro-D-$Phe^4$-$Lys^5$-$Val^6$-$Cys^7$)-$Thr^8$-$NH_2$•2 $CH_3COOH$.

HPLC : tr = 9,6 min

$^1$H NMR (400 Mhz, $D_2O$) : δ 0,64-0,76 (m, 1H); 0,78-0,90 (m, 1H); 0,91 (d, J = 6,7, 3H); 0,94 (d, J = 6,6, 3H); 1,19 (d, J = 6,5, 3H); 1,35-1,47 (M, 3H); 1,72-1,82 (m, 1H); 1,89 (s, 6H); 2,11- 2,21 (m, 1H); 2,47 (dd, J = 14,6 , J = 4,0, 1H); 2,59 (dd, J = 14,7, J = 9,8, 1H); 2,69 (dd, J = 14,7, J = 3,9, 1H); 2,72-2,81 (M, 3H); 2,84 (d, J = 8,0, 2H); 2,94 (d, J = 2,9, 1H); 3,00 (s, 1H); 3,30 (dd, J = 13,6 , J = 9,0, 1H); 3,42 (dd, J = 13,7 , J = 5,7, 1H); 3,99 (M, 2H); 4,21 (dd, J = 6,6 , J = 4,0, 1H); 4,26-4,34 (M, 3H); 4,59 (t, J = 7,6, 1H); 4,82-4,92 (M, 3H); 6,75 (d, J = 8,5, 2H); 7,05 (d, J = 8,5, 2H); 7,35 (d, J = 8,7, 2H); 7,46 (dd, J = 8,6, J = 1,2, 1H); 7,50-7,56 (M, 2H); 7,78 (ls, 1H); 7,84-7,88 (m, 1H); 7,88-7,94 (M, 2H).

HRMS ($H_2O$) m/z : 1102,4476 [M+H]+ (calc. 1102,4490) C52 H68 N11 O12 S2

***Exemple 21 :*** H-D-2-$Nal^1$-cyclo($Cys^2$-$Tyr^3$-*p*-Ph-D-$Phe^4$-$Lys^5$-$Val^6$-$Cys^7$)-$Thr^8$-$NH_2$•2 $CH_3COOH$

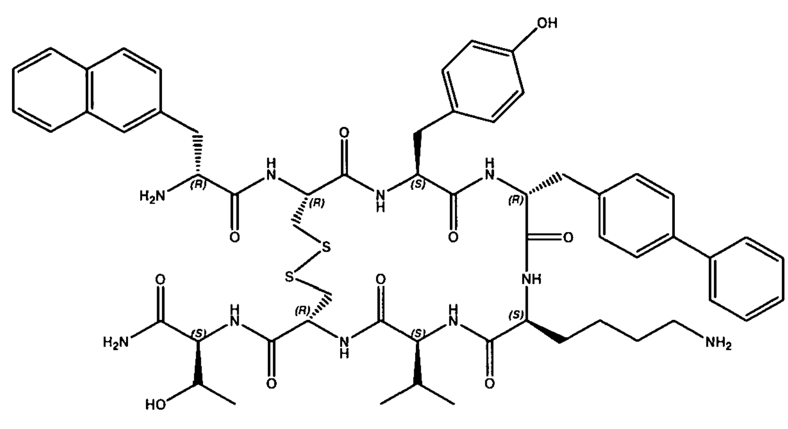

Le peptide ouvert est obtenu selon le protocole décrit précédemment par la succession de déprotection du groupement Fmoc (9-fluorènylméthyloxycarbonyl) et couplage de l'acide aminé suivant sur support solide dans l'ordre suivant : Fmoc-L-Thr(tBu)-OH puis Fmoc-L-Cys(Trt)-OH puis Fmoc-L-Val-OH puis Fmoc-L-Lys(Boc)-OH puis Fmoc-*p*-Ph-D-Phe-OH puis Fmoc-L-Tyr(tBu)-OH puis Fmoc-L-Cys(Trt)-OH et enfin Boc-β-(2-naphthyl)-D-Ala-OH.

Le composé est cyclisé par la formation d'un pont disulfure, puis clivé de la résine, purifié, et enfin obtenu sous forme d'acétate selon le mode opératoire décrit précédemment pour conduire au composé H-D-2-$Nal^1$-cyclo

(Cys[2]-Tyr[3]-*p*-Ph-D-Phe[4]-Lys[5]-Val[6]-Cys[7])-Thr[8]-$NH_2$•2 $CH_3COOH$.

HPLC : tr = 9,9 min

$^1$H NMR (400 Mhz, $D_2O$) : δ 051-062 (m, 1H); 0,62-0,72 (m, 1H); 0,90 (d, J = 6,6, 3H); 0,93 (d, J = 6,6, 3H); 1,20 (d, J = 6,3, 3H); 1,23-1,28 (m, 1H); 1,28-1,36 (m, 1H); 1,62-1,73 (m, 1H); 1,89 (s, 6H); 2,12-2,24 (m, 1H); 2,35-2,46 (M, 3H); 2,54 (dd, J = 14,6, J = 9,5, 1H); 2,67-2,82 (M, 2H); 2,82-2,98 (M, 4H); 3,20 (dd, J = 13,3, J = 9,2, 1H); 3,36 (dd, J = 13,4, J = 5,6, 1H); 3,91 (dd, J = 10,9, J = 3,4, 1H); 3,96 (d, J = 9,8, 1H); 4,15 (dd, J = 8,2, J = 6,2, 1H); 4,20-4,30 (M, 2H); 4,32 (d, J = 3,9, 1H); 4,31 (dd, J = 7,7, J = 7,2, 1H); 4,84-4,90 (M, 2H); 6,81 (d, J = 6,8, 2H); 7,09 (d, J = 8,4, 2H); 7,25 (d, J = 7,9, 2H); 7,41-7,46 (M, 2H); 7,48-7,58 (M, 4H); 7,66 (d, J = 8,0, 2H); 7,71 (d, J = 7,4, 2H); 7,75 (ls, 1H); 7,83-7,87 (m, 1H); 7,87-7,92 (M, 2H).

HRMS ($H_2O$) m/z : 1133,5002 [M+H]+ (calc. 1133,4953) C58 H73 N10 O10 S2

***Exemple 22 :*** H-D-2-Nal[1]-cyclo(Cys[2]-Tyr[3]-*p*-2-(2-méthoxyéthoxy)éthoxy)-D-Phe[4]-Lys[5]-Val[6]-Cys[7])-Thr[8]-$NH_2$]•2 $CH_3COOH$

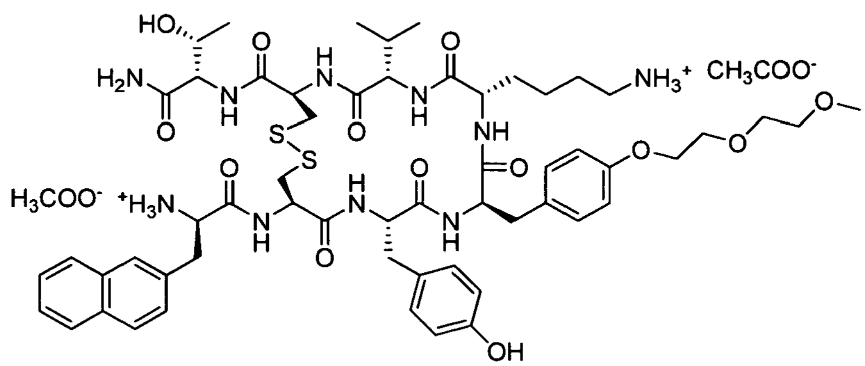

Le peptide ouvert est obtenu selon le protocole décrit précédemment par la succession de déprotection du groupement Fmoc (9-fluorènylméthyloxycarbonyl) et couplage de l'acide aminé suivant sur support solide dans l'ordre suivant : Fmoc-L-Thr(tBu)-OH puis Fmoc-L-Cys(Trt)-OH puis Fmoc-L-Val-OH puis Fmoc-L-Lys(Boc)-OH puis Fmoc-*p*-2-(2-méthoxyéthoxy)éthoxy)-D-Phe-OH puis Fmoc-L-Tyr(tBu)-OH puis Boc-L-Cys(Trt)-OH et enfin Fmoc-β-(2-naphthyl)-D-Ala-OH.

Le composé est cyclisé par la formation d'un pont disulfure, puis clivé de la résine, purifié, et enfin obtenu sous forme d'acétate selon le mode opératoire décrit précédemment pour conduire au composé H-D-2-Nal[1]-cyclo (Cys[2]-Tyr[3]-*p*-2-(2-méthoxyéthoxy)éthoxy)-D-Phe[4]-Lys[5]-Val[6]-Cys[7])-Thr[8]-$NH_2$]•2 $CH_3COOH$.

$^1$H NMR (400 MHz, $D_2O$) : δ 0,64-0,76 (m, 1H); 0,76-0,88 (m, 1H); 0,93 (d, J = 6,9, 3H); 0,96 (d, J = 6,9, 3H); 1,23 (d, J = 6,3, 3H); 1,35-1,52 (M, 2H); 1,72-1,82 (m, 1H); 1,91 (s, 6H); 2,13-2,24 (m, 1H); 2,43 (dd, J = 14,6, J = 4,8, 1H); 2,54 (dd, J = 14,5, J = 9,3, 1H); 2,70-2,95 (M, 4H); 3,19 (dd, J = 13,1, J = 9,6, 1H); 3,36 (s, 3H); 3,60 (t, J = 3,4, 1H); 3,61 (t, J = 4,4, 1H); 3,71 (t, J = 4,4, 1H); 3,72 (t, J = 3,4, 1H); 3,84-3,89 (M, 2H); 3,99 (d, J = 10,8, 1H); 4,00 (d, J = 4,01, 1H); 4,07-4,14 (m, 1H); 4,16-4,20 (M, 2H); 4,23-4,30 (M, 2H); 4,35 (d, J = 3,9, 1H); 4,60 (dd, J = 7,6, J = 6,8, 1H); 4,83-4,88 (M, 2H); 6,82 (d, J = 8,4, 2H); 6,96 (d, J = 8,6, 2H); 7,07 (d, J = 8,4, 2H); 7,13 (d, J = 8,6, 2H); 7,46 (dd, J = 8,5 , J = 1,3, 1H); 7,51-7,56 (M, 2H); 7,76 (ls, 1H); 7,85-7,94 (M, 3H).

***Exemple 23 :*** H-2-Nal[1]-cyclo(D-Cys[2]-D-Tyr[3]-Trp[4]-D-Lys[5]-D-Val[6]-D-Cys[7])-D-Thr[8]-$NH_2$•2 $CH_3COOH$

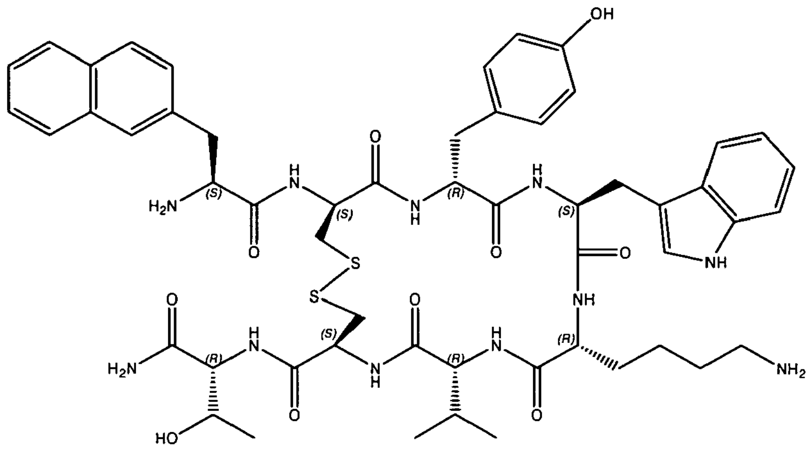

Le peptide ouvert est obtenu selon le protocole décrit précédemment par la succession de déprotection du groupement Fmoc (9-fluorènylméthyloxycarbonyl) et couplage de l'acide aminé suivant sur support solide dans l'ordre suivant : Fmoc-D-Thr(tBu)-OH puis Fmoc-D-Cys(Trt)-OH puis Fmoc-D-Val-OH puis Fmoc-D-Lys(Boc)-OH puis Fmoc-L-Trp-OH puis Fmoc-D-Tyr(tBu)-OH puis Fmoc-D-Cys(Trt)-OH et enfin Boc-β-(2-naphthyl)-L-Ala-OH.

Le composé est cyclisé par la formation d'un pont disulfure, puis clivé de la résine, purifié, et enfin obtenu sous forme d'acétate selon le mode opératoire décrit précédemment pour conduire au composé H-2-$Nal^1$-cyclo(D-$Cys^2$-D-$Tyr^3$-$Trp^4$-D-$Lys^5$-D-$Val^6$-D-$Cys^7$)-D-$Thr^8$-$NH_2$•2 $CH_3COOH$.

HPLC : tr = 9.87 min

$^1H$ NMR (400 MHz, $D_2O$) : δ 0.10-0.22 (m, 1H), 0.32-0.46 (m, 1H), 0.90 (d, J = 7,6, 3H), 0.91 (d, J = 7,3, 3H), 1,10-1,30 (M, 4H) 1,19 (d, J = 6,2, 3H), 1,50-1,62 (m, 1H), 1.95 (s, 6H), 2,10-2,20 (m, 1H), 2,45 (dd, J = 14,5, J = 3,9, 1H), 2,52-2,64 (M, 3H), 2,70 (dd, J = 14,8, J = 3,8, 1H), 2,78 (dd, J = 14,3, J = 11,7, 1H), 2,90-3,60 (M, 4H), 3,35 (dd, J = 13,5, J = 9,0, 1H), 3,46 (dd, J = 14,0, J = 6,0, 1H), 3,81 (dd, J = 10,9, J = 3,4, 1H), 3,95 (d, J = 9,4, 1H), 4,18-4,27 (M, 2H), 4,31 (d, J = 3,6, 1H), 4,42 (dd, J = 8,5 , J = 5,9, 1H), 4,64 (t, J = 7,4, 1H), 4,83-4,92 (M, 2H), 6,84 (d, J = 8,6, 2H), 7,12 (d, J = 8,6, 2H), 7,16 (t, J = 7,1, 1H), 7,22 (t, J = 7,1, 1H), 7,43-7,49 (M, 2H), 7,50-7,57 (M, 3H), 7,77 (ls, 1H), 7,83-7,88 (m, 1H), 7,90 (ld, J = 8,9, 2H).

HRMS ($H_2O$) m/z : 1096,4749 [M+H]+

## 2. Etude des composés selon l'invention

### 2.1 Activité des cmposés octapetidiques sur les récepteurs à la somatostatine

**2.1.1** *Protocole de mesure de l'affinité des peptides sur le récepteurs de la somatostatine*

L'affinité des composés de l'invention pour les récepteurs de la Somatostatine est déterminée par la mesure de l'inhibition de la liaison de [125I-Tyr11]SRIF-14 à des préparations membranaires de cellules CHO-K1 transfectées.

Les cellules CHO-K1 exprimant de façon stable chacun des sous types de récepteurs de la somatostatine sont collectées avec 0,5 mM d'EDTA et centrifugées à 500 g pendant 5 minutes à 4°C. Le culot est re-suspendu dans tampon phosphate (PBS) et centrifugé à 500 g pendant 5 minutes à 4°C. Le culot est re-suspendu dans du tampon Tris 50 mM à pH 7,4 et centrifugé à 500 g pendant 5 minutes à 4°C. Les cellules sont lysées par sonication et centrifugées à 39 000 g pendant 10 minutes. Le culot est re-suspendu dans du tampon Tris 50 mM à pH 7,4, un aliquote est prélevé pour le dosage de protéines et le reste est centrifugé à 50 000 g pendant 10 minutes. Les membranes obtenues dans ce dernier culot sont stockées à -80°C.

La mesure de l'inhibition compétitive de la liaison de la [125I-Tyr11]SRIF-14 (Perkin Elmer) sur chacun des sous types de récepteurs de la somatostatine est effectuée en duplicats dans des plaques 96 puits en polypropylène. Les membranes cellulaires (5 à 20 μg de protéines/puits) sont incubées avec la [125I-Tyr11]SRIF-14 (0,05 à 0.1 nM) pendant 50 à 90 minutes à 37°C (conditions dépendantes du sous-type de récepteur) dans un milieu tampon HEPES 50 mM pH 7,4, comprenant 0,2 % d'albumine bovine de sérum (BSA), MgCl2 5 mM, Trasylol 200 KIU/mL, Bacitracine 0,02 mg/mL, phénylméthylsulfonyl fluoride 0,02 mg/mL.

La [125I-Tyr11]SRIF-14 liée est séparée de la [125I-Tyr11]SRIF-14 libre par filtration à travers des plaques de fibre de verre GF/C (Unifilter, Perkin Elmer) pré-imprégnées avec 0,1 % de polyéthylènimine (P.E.I.), en utilisant un Filtermate 96 (Perkin Elmer). Les filtres sont lavés avec du tampon Tris-HCl 50 mM, pH 7,4 à 4°C et la radioactivité

présente est déterminée à l'aide d'un compteur (TopCount, Perkin Elmer).

Les données sont analysées par régression non linéaire assistée par le logiciel XLfit 4.2 (IDBS).

**2.1.2** *Résultats*

Les exemples 1, 2, 3, 4, 5, 6, 8, 9, 10, 11, 12, 13, 14, 15, 16, 17, 18, 19, 20 et 21 ont une affinité sur le sous-type 2 des récepteurs à la somatostatine inférieure ou égale à 5 μM.

Les exemples 1, 2, 3, 5, 6, 8, 9, 10, 11, 12, 13, 14, 15, 16, 17, 18, 19, 20 et 21 ont une affinité sur le sous-type 2 des récepteurs à la somatostatine inférieure ou égale à 700 nM.

Les exemples 1, 2, 6, 8, 9, 11, 12, 13, 14, 15, 16, 17, 18, 19, 20 et 21 ont une affinité sur le sous-type 2 des récepteurs à la somatostatine inférieure ou égale à 50 nM.

Les exemples 1, 6, 12, 13, 14, 15, 16, 17, 18, 19 et 20 ont une affinité sur le sous-type 2 des récepteurs à la somatostatine inférieure ou égale à 5 nM.

Les exemples 1, 2, 3, 5, 6, 8, 9, 10, 11, 12, 13, 14, 15, 16, 17, 18, 19, 20 et 21 ont une affinité sur le sous-type 5 des récepteurs à la somatostatine inférieure ou égale à 5 μM.

Les exemples 1, 2, 6, 8, 9, 11, 12, 13, 14, 15, 16, 17, 18, 19, 20 et 21 ont une affinité sur le sous-type 5 des récepteurs à la somatostatine inférieure ou égale à 1 μM.

Les exemples 1, 6, 8, 9, 12, 15, 17, 18, 19 et 21 ont une affinité sur le sous-type 5 des récepteurs à la somatostatine inférieure ou égale à 100 nM.

## 2.2 Propriétés Physico-chimiques des composés octapetidiques

Les peptides obtenus font l'objet d'une étude de leurs propriétés d'auto-assemblage.

La procédure utilisée comporte trois étapes : la mise en solution des peptides et analyse macroscopique ; les analyses spectroscopiques et microscopiques des structures formées et les analyses par diffraction de rayons X aux petits angles (SAXS).

**2.2.1** *Mise en solution des peptides et analyse macroscopique*

La première étape de la caractérisation des dérivés est la réalisation d'une gamme de concentrations de 3 à 15 % en masse de peptide dans l'eau. Une gélification de la solution indique la présence de structures auto-assemblées qui peuvent être des nanotubes mais aussi des fibres ou d'autres structures auto-organisées.

**2.2.2** *Analyses spectroscopiques et microscopiques des structures formées*

Cette étape a pour but de vérifier la présence de structures auto-assemblées, de caractériser leur auto-assemblage et de les visualiser par microscopie.

La spectroscopie infrarouge permet d'obtenir des informations sur le mode d'empilement des peptides dans les auto-assemblages. Le spectre de la bande amide, reflétant les spectres d'absorption des fonctions carbonyles du squelette peptidique, donne des informations sur le type de liaison hydrogène dans lequel ces groupements sont impliqués (liaisons H conduisant à la formation de feuillet β, d'hélice α ou encore de boucle ou de conformation aléatoire) et en conséquence sur la structure secondaire et tertiaire des peptides en solution et auto-assemblés. La technique par réflexion totale atténuée (ATR-FTIR) utilise un cristal sur lequel est déposé l'échantillon, solide ou liquide. La lumière infrarouge est guidée à travers ce cristal selon un angle lui permettant de se réfléchir plusieurs fois à l'intérieur du cristal, ce qui augmente la sensibilité de la technique. Grâce aux performances de cet appareil, il est alors possible de faire des mesures sur des solutions relativement diluées. La pénétration du faisceau dans l'échantillon déposé sur le cristal se fait sur quelques microns, ce qui fait que seules les molécules/structures qui se déposent sur la surface du cristal sont analysées. De plus, cette technique est non destructive.

Les structures assemblées sont ensuite visualisées par microscopie électronique par transmission (MET). Cette technique très puissante, permettant des grossissements supérieurs à 500 000 fois, est l'une des rares techniques capables de visualiser des structures de taille nanométrique. L'échantillon est soumis à un faisceau d'électrons. La visualisation du faisceau transmis se fait soit par un écran photoluminescent, soit par une caméra CCD. L'échantillon dans le microscope étant sous vide, ce sont des échantillons séchés après coloration par des sels d'uranyl pour augmenter le contraste qui sont observés.

Le peptide en solution est d'abord déposé sur une grille en cuivre recouverte d'un film de carbone. Après dépôt du matériel, l'excès de liquide est éliminé. De la même façon, la solution de colorant, dans notre cas de l'acétate d'uranyle, est déposée sur la grille, l'excès est éliminé et la grille est séchée à l'air libre. Elle peut alors être introduite dans le microscope.

**2.2.3** *Analyses par diffraction de rayons X aux petits angles (SAXS)*

Les échantillons, liquides ou gels, sont introduits dans un capillaire et soumis à un flux monochromatique de rayons X. L'intensité diffusée est mesurée en fonction de l'angle de diffusion par rapport à l'angle incident.

Les diagrammes de diffusion ainsi obtenus doivent faire l'objet d'une corrélation avec un modèle théorique proche des structures présentes dans l'échantillon. Dans les cas où les peptides s'auto-assemblent sous forme de nanotubes creux, la modélisation des données expérimentales avec la diffusion théorique produite par des colonnes creuses infiniment longues permet de déterminer avec précision le diamètre des colonnes et d'estimer l'épaisseur de leur paroi (Valéry et al. dans Biophysical journal, 2004, 86(4), 2484-2501 et Proc. Natl. Acad. Sci., 2003, 100(18) 10258-10262). Pour des auto-assemblages polydisperses, nanotubulaires, fibrilaires, micellaires ou lamellaires, c'est la corrélation des données spectroscopiques, microscopiques et de diffusion de RX qui permet d'estimer une gamme de taille pour les objets observés.

**2.2.4** *Résultats*

Les résultas d'analyse des propriétés physico-chimiques des composés sont présentés dans le tableau 1 ci-dessous :

*Tableau 1*

| Exemple | Assemblage |
|---|---|
| 1 | nanotube 20 nm monodisperse |
| 2 | nanotube 12 nm monodisperse |
| 3 | nanotube 9,3 nm monodisperse |
| 4 | fibres |
| 5 | soluble |
| 6 | nanotube 35 nm monodisperse |
| 7 | insoluble |
| 8 | insoluble |
| 9 | nanotube 22 nm monodisperse |
| 10 | fibres |
| 11 | soluble |
| 12 | nanotube 17 nm monodisperse |
| 13 | nanotube 17 nm monodisperse |
| 14 | nanotube 18 nm monodisperse |
| 15 | nanotube 15,6 nm monodisperse |
| 16 | nanotube 17 nm monodisperse |
| 17 | nanotube 15,3 nm monodisperse |
| 18 | nanotube 18,4-18,8 nm monodisperse |
| 19 | nanotube environ 29 nm polydisperse |
| 20 | nanotube 17,6 nm monodisperse |
| 21 | insoluble |
| 22 | nanotube 17,4 nm monodisperse |

Certains peptides permettent d'obtenir des nanotubes monodisperses, alors que d'autres s'assemblent en fibres ou nanotubes polydisperses. Enfin, quelques uns sont solubles,
ou au contraire insolubles dans les conditions opératoires mentionnées ci-dessus.

Les dérivés s'auto-assemblant en nanotubes monodisperses de peptides sont les suivants: exemples 1, 2, 3,

6, 9, 12, 13, 14, 15, 16, 17, 18, et 20. Une gamme de diamètres de 9,3 à 35 nm est obtenue.

Le dérivé de l'exemple 19 s'assemble en nanotubes de diamètres polydisperses.

Les dérivés permettant d'obtenir des fibres auto-assemblées sont les suivants : exemples 4 et 10.

Les dérivés solubles aux concentrations étudiées sont les suivants : exemples 5 et 11.

Enfin, les dérivés insolubles aux concentrations étudiées sont les suivants : exemples 7, 8 et 21.

Une étude des composés dans d'autres gammes de concentrations pourrait révéler une évolution de ces comportements.

**2.3 Rhéologie des composés octapeptidiques**

Les propriétés visqueuses et viscoélastiques des solutions/gels des composés selon l'invention peuvent être étudiées par rhéologie selon les modes opératoires décrits ci-après.

**2.3.1** *Équipement et conditions de mesures*

Les mesures sont en général réalisées en utilisant un rhéomètre de type « Control Stress Haake 600 » (Haake, Allemagne) équipé d'une géométrie de mesure cône/plan, avec un cône de 20mm de diamètre, un angle de 1 degré et un entrefer de 52 microns. La température de mesure est fixée à 25°C +/- 0.2°C à l'aide d'un bain thermostaté mais peut être modulée si nécessaire.

***2.3.2*** *Types de mesures et modes opératoires*

Deux types complémentaires de tests sont effectués, d'une part des mesures en mode statique et d'autre part des mesures en mode dynamique (rhéologie oscillatoire), toutes deux décrites brièvement ci-après.

*Mode statique*

Une courbe d'écoulement est enregistrée en mode statique (une contrainte de cisaillement $\tau$ (Pa) en fonction du taux de cisaillement $\gamma$ ($s^{-1}$)). Cette courbe permet d'accéder à des informations telles que le comportement de flux, la viscosité, la contrainte limite d'écoulement et une éventuelle thixotropie des échantillons. Les solutions/gels présentant des nanostructures peptidiques de type nanotubes ou fibres se comportent en général comme des fluides non Newtoniens. En adaptant à la courbe de flux expérimentale des modèles théoriques tels que ceux de Cross (Cross MM. J Colloid Sci 1965 20 ; 417-37) ou de Casson (Casson, N. In Rheology of Disperse Systems, C.C. Mill ed, 1959, p. 82, Pergamon Press, New York, NY.), il est possible d'accéder par extrapolation aux valeurs de viscosité infinie ($\eta\infty$) et viscosité 0, $\eta_0$ (Pa.s), servant de paramètres de comparaison et de référence pour définir la viscosité de ces solutions/gels.

*Mode dynamique (rhéologie oscillatoire)*

Pour les mesures dynamiques, une déformation de cisaillement (strain ) ($\gamma_0$) (Pa) de type sinusoïdal, d'amplitude et de fréquence connue est appliqué à l'échantillon. La contrainte de cisaillement sinusoïdale résultante est mesurée (amplitude $\tau_0$). Les solutions/gels présentant des nanostructures peptidiques de type nanotubes ou fibres sont généralement des systèmes viscoélastiques. Dans ce cas, les sinusoïdes enregistrées pour la déformation de cisaillement (strain) et la contrainte de cisaillement (stress) présentent un déphasage d'un angle $\delta$. Les valeurs de $\tau_0$. $\gamma_0$ et $\delta$ permettent d'accéder aux paramètres viscoélastiques :

Le module complexe $\left|G^*\right| = \dfrac{\tau_0}{\gamma_0}$

Le module de conservation $G' = |G^*| \cos\delta$

Le module de perte $G'' = |G^*| \sin\delta$

Le module de conservation (aussi appelé module élastique) est une mesure de l'énergie élastique emmagasinée (ou restituée) par unité de volume, alors que le module de perte est une mesure de l'énergie dissipée par unité de volume. Ces modules doivent être calculés dans la région viscoélastique linéaire (i.e. conditions expérimentales pour lesquelles ces modules sont indépendants de l'amplitude de la contrainte de cisaillement (strain)). Ces conditions expérimentales sont en général déterminées par une expérience dite de « stress sweep » qui consiste en une mesure du module élastique et du module de perte en fonction de l'amplitude de la déformation imposée (pour une fréquence donnée, typiquement 1Hz). L'allure générale est que, pour les faibles déformations, G' et G" varient très peu avec la

déformation. Puis, au passage au seuil d'écoulement, on observe une forte diminution de G' et un maximum de G". Cette transition délimite la région de viscoélasticité linéaire. Lorsque la région de viscoélasticité linéaire est connue, d'autres tests peuvent être effectués tels que des mesures de « frequency sweep » consistant à mesurer la dépendance de G' et G" en fonction de la fréquence de cisaillement à une amplitude de déformation fixe, comprise dans la région de viscoélasticité linéaire.

Des essais de fluage et de récupération peuvent également être réalisés pour caractériser les solutions/gels des composés. Dans ce type de test une contrainte d'amplitude constante à l'instant initial t=0 est imposée à l'échantillon, contrainte qui est maintenue constante au cours du temps. La viscosité zero, $\eta_0$, est alors égale à l'inverse de la pente de la courbe de la complaisance élastique (J) en fonction du temps.

**2.4 Etude de la libération des composés octapetidiques**

Des études de libération peuvent être menées sur les composés octapeptidiques selon l'invention sous forme gel, solide, ou en suspension. Deux types de tests sont envisageables : des tests de libération statiques et/ou des tests dynamiques, tous deux décrits brièvement ci-après.

Les paramètres expérimentaux sont communs aux deux types de tests. Le milieu de libération est un milieu liquide physico-chimiquement bien contrôlé, préférablement un tampon PBS (phosphate buffer saline) ajusté à pH 7.4 et maintenu à 37˚C. La courbe de libération est définie comme la concentration du composé étudié dans le milieu de dissolution (exprimée en % de composé libéré) en fonction du temps (minutes, heures, jours). Pour initier les tests, une quantité connue d'échantillon est placée dans le milieu de dissolution à temps 0 (t=0 min/h/j), l'échantillon étant éventuellement conditionné dans un dispositif adapté à son état physique initial (gel, solide, suspension). Les mesures de concentration du composé dans le milieu de libération sont réalisées par une ou plusieurs méthodes analytiques bien connues de l'état de l'art comme la spectrophotométrie UV-visible ou la HPLC (Chromatographie Liquide à Haute Performance). Les paramètres des méthodes analytiques sont ajustés en fonction des caractéristiques physico-chimiques des composés.

***2.4.1** Tests statiques*

Les études de libération en mode statique sont conduites sans soumettre les échantillons à une agitation contrôlée ou à un flux de milieu de libération. Les prélèvements de milieu de libération et / ou les déterminations de concentration ne sont généralement pas automatisés et requièrent des opérations manuelles. Par exemple, certaines études statiques de libération peuvent être réalisées sur des échantillons correspondant à de l'ordre de 2 à 5mg de composé pur dans 20mL de PBS (typiquement dans des béchers de 50mL) maintenus dans une étuve à 37˚C. Les mesures de concentration sont réalisées sur des prélèvements représentatifs du milieu de libération effectués à des temps choisis.

***2.4.2** Tests dynamiques*

Pour les études dynamiques de libération, une agitation contrôlée ou un flux de milieu de libération est appliqué aux échantillons. Dans ce mode, les prélèvements et / ou déterminations de concentration sont généralement automatisés et les études peuvent être conduites dans des systèmes / instruments standardisés et bien connus de l'état de l'art (cf. systèmes de la Pharmacopée Européenne). Par exemple, certaines études en mode dynamique peuvent être réalisées sur des échantillons correspondant à de l'ordre de 10mg de composé pur placés dans 100mL de PBS préalablement introduits dans les béchers standardisés et maintenus à 37˚C. L'agitation et les prélèvements / mesures sont contrôlés par un instrument d'essai de dissolution de type appareil 1 ou 2 décrit par la Pharmacopée Européenne.

**Revendications**

**1.** Composé octapeptidique de formule générale (I)

$$H\text{-}2\text{-}Nal^1\text{-cyclo}(Cys^2\text{-}Tyr^3\text{-}AA^4\text{-}Lys^5\text{-}Val^6\text{-}Cys^7)\text{-}Thr^8\text{-}NH_2 \qquad (I)$$

dans laquelle $AA^4$ représente un acide aminé de formule

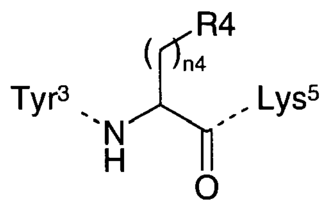

dans laquelle n4 représente un entier de 0 à 3 et R4 représente un atome d'hydrogène, un radical alkyle, aryle ou hétéroaryle, les radicaux aryle et hétéroaryle étant éventuellement substitués par un ou plusieurs radicaux identiques ou différents choisis parmi halo, nitro, hydroxy, aryle, OR41 ;
R41 représente un radical

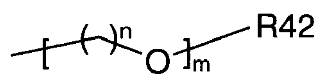

dans lequel R42 représente un radical
alkyle ou un atome d'hydrogène, n est un entier de 2 à 4, et m est un entier de 1 à 4 ; étant entendu que tous les acides aminés peuvent être de configuration D ou L,
à l'exclusion des composés H-D-2-Nal$^1$-cyclo(Cys$^2$-Tyr$^3$-β-(3-pyridyl)-D-Ala$^4$- Lys$^5$-Val$^6$-Cys$^7$)-Thr$^8$-$NH_2$, H-D-2-Nal$^1$-cyclo(Cys$^2$-Tyr$^3$-D-Phe$^4$-Lys$^5$-Val$^6$-Cys$^7$)-Thr$^8$-$NH_2$, H-D-2-Nal$^1$-cyclo(Cys$^2$-Tyr$^3$-D-Trp$^4$-Lys$^5$-Val$^6$-Cys$^7$)-Thr$^8$-$NH_2$ et de leurs sels,
ou un sel pharmaceutiquement acceptable de ce composé.

**2.** Composé octapeptidique de formule générale (I) selon la revendication 1 **caractérisé en ce que** n4 représente un entier de 0 à 2 et R4 représente un radical alkyle, aryle ou hétéroaryle, les radicaux aryle et hétéroaryle étant éventuellement substitués par un ou plusieurs radicaux identiques ou différents choisis parmi halo, nitro, hydroxy, aryle, OR41 ;
R41 représente un radical

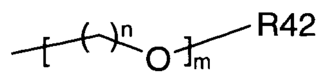

dans lequel R42 représente un radical alkyle, n et m représentent 2.

**3.** Composé octapeptidique de formule générale (I) selon l'une des revendications 1 ou 2 dans laquelle le terme aryle représente un radical choisi parmi phényle, naphtyle, anthryle et fluorényle.

**4.** Composé octapeptidique de formule générale (I) selon l'une des revendications 1 à 3 dans laquelle le terme hétéroaryle représente un radical choisi parmi pyridinyle, pyrimidinyle, furyle, thiènyle, benzothiènyle, oxazolyle, benzoxazolyle, isoxazolyle, thiazolyle, pyrolyle, pyrazolyle, imidazolyle, triazolyle, tétrazolyle.

**5.** Composé octapeptidique de formule générale (I) selon l'une des revendications 1 à 4 dans laquelle le terme hétéroaryle représente un radical choisi parmi thiènyle, benzothiènyle et imidazolyle.

**6.** Composé octapeptidique de formule générale (I) selon l'une des revendications 1 ou 5 dans laquelle le terme alkyle représente un radical choisi parmi méthyle, éthyle, propyle, isopropyle, butyle, isobutyle, tert-butyle.

**7.** Composé octapeptidique de formule générale (I) selon la revendication 1 dans laquelle AA$^4$ représente un acide aminé choisi parmi Trp, Ala, β-(3-benzothiényl)-Ala, β-(2-thiényl)-Ala, β-(9-anthryl)-Ala, β-(2-fluorenyl)-Ala, His, Val, 1-Nal, 2-Nal, phényl-Gly, Homo-Phe, *p*-Br-Phe, *p*-F-Phe, *m*-F-Phe, *o*-F-Phe, *m*-Br-Phe, *o*-Br-Phe, *p*-$NO_2$-Phe, 3,5-

difuoro-Phe, 4-phényl-Phe, Tyr, *p*-(2-(2-méthoxyéthoxy)éthoxy)-Phe.

**8.** Composé octapeptidique de formule générale (I) selon l'une des revendications 1 à 7 **caractérisé en ce que** l'acide aminé AA$^4$ est de configuration D.

**9.** Composé octapeptidique de formule générale (I) selon la revendication 8 **caractérisé en ce que** les acides aminés 2-Nal$^1$ et AA$^4$ sont de configuration D, les autres acides aminés étant de configuration L.

**10.** Composé octapeptidique de formule générale (I) selon l'une des revendications 1 à 7 **caractérisé en ce que** l'acide aminé AA$^4$ est de configuration L.

**11.** Composé octapeptidique de formule générale (I) selon la revendication 10 **caractérisé en ce que** les acides aminés 2-Nal$^1$ et AA$^4$ sont de configuration L, les autres acides aminés étant de configuration D.

**12.** Composé octapeptidique de formule générale (I) selon l'une des revendications 1 à 7 **caractérisé en ce qu'**il s'agit d'un composé choisi parmi :

H-D-2-Nal$^1$-cyclo(Cys$^2$-Tyr$^3$-β-(3-benzothienyl)-D-Ala$^4$-Lys$^5$-Val$^6$-Cys$^7$)-Thr$^8$-NH2
H-D-2-Nal$^1$-cyclo(Cys$^2$-Tyr$^3$-β-(2-thienyl)-D-Ala$^4$-Lys$^5$-Val$^6$-Cys$^7$)-Thr$^8$-NH2
H-D-2-Nal$^1$-cyclo(Cys$^2$-Tyr$^3$-D-His$^4$-Lys$^5$-Val$^6$-Cys$^7$)-Thr$^8$-NH$_2$
H-D-2-Nal$^1$-cyclo(Cys$^2$-Tyr$^3$-D-His$^4$-Lys$^5$-Val$^6$-Cys$^7$)-Thr$^8$-NH$_2$
H-D-2-Nal$^1$-cyclo(Cys$^2$-Tyr$^3$-D-His$^4$-Lys$^5$-Val$^6$-Cys$^7$)-Thr$^8$-NH$_2$
H-D-2-Nal$^1$-cyclo(Cys$^2$-Tyr$^3$-β-(1-naphthyl)-D-Ala$^4$-Lys$^5$-Val$^6$-Cys$^7$)-Thr$^8$-NH$_2$
H-D-2-Nal$^1$-cyclo(Cys$^2$-Tyr$^3$-β-(2-naphthyl)-D-Ala$^4$-Lys$^5$-Val$^6$-Cys$^7$)-Thr$^8$-NH$_2$
H-D-2-Nal$^1$-cyclo(Cys$^2$-Tyr$^3$-β-(9-anthryl)-D-Ala$^4$-Lys$^5$-Val$^6$-Cys$^7$)-Thr$^8$-NH$_2$
H-D-2-Nal$^1$-cyclo(Cys$^2$-Tyr$^3$-β-(2-fluorenyl)-D-Ala$^4$-Lys$^5$-Val$^6$-Cys$^7$)-Thr$^8$-NH$_2$
H-D-2-Nal$^1$-cyclo(Cys$^2$-Tyr$^3$-Ph-D-Gly$^4$-Lys$^5$-Val$^6$-Cys$^7$)-Thr$^8$-NH$_2$
H-D-2-Nal'-cyclo(Cys$^2$-Tyr$^3$-homo-D-Phe$^4$-Lys$^S$-Val$^s$-Cys')-Thr$^B$-NH$_2$
H-D-2-Nal'-cyclo(Cys2-Tyr3-*p*-Br-D-Phe4-LysS-Vals-Cys')-ThrB-NH2
H-D-2-Nal$^1$-cyclo(Cys$^2$-Tyr$^3$-*p*-F-D-Phe$^4$-Lys$^5$-Val$^6$-Cys$^7$)-Thr$^8$-NH$_2$
H-D-2-Nal$^1$-cyclo(Cys$^2$-Tyr$^3$-D-Tyr$^4$-Lys$^5$-Val$^6$-Cys$^7$)-Thr$^8$-NH$_2$
H-D-2-Nal$^1$-cyclo(Cys$^2$-Tyr$^3$-*m*-F-D-Phe$^4$-Lys$^5$-Val$^6$-Cys$^7$)-Thr$^8$-NH$_2$
H-D-2-Nal$^1$-cyclo(Cys$^2$-Tyr$^3$-*o*-F-D-Phe$^4$-Lys$^5$-Val$^6$-Cys$^7$)-Thr$^8$-NH$_2$
H-D-2-Nal$^1$-cyclo(Cys$^2$-Tyr$^3$-*3,5*-diF-D-Phe$^4$-Lys$^5$-Val$^6$-Cys$^7$)-Thr$^8$-NH$_2$
H-D-2-Nal$^1$-cyclo(Cys$^2$-Tyr$^3$-*m*-Br-D-Phe$^4$-Lys$^5$-Val$^6$-Cys$^7$)-Thr$^8$-NH$_2$
H-D-2-Nal$^1$-cyclo(Cys$^2$-Tyr$^3$-*o*-Br-D-Phe$^4$-Lys$^5$-Val$^6$-Cys$^7$)-Thr$^8$-NH$_2$
H-D-2-Nal$^1$-cyclo(Cys$^2$-Tyr$^3$-*p*-Nitro-D-Phe$^4$-Lys$^5$-Val$^6$-Cys$^7$)-Thr$^8$-NH$_2$
H-D-2-Nal$^1$-cyclo(Cys$^2$-Tyr$^3$-*p*-Ph-D-Phe$^4$-Lys$^5$-Val$^6$-Cys$^7$)-Thr$^8$-NH$_2$
H-D-2-Nal$^1$-cyclo(Cys$^2$-Tyr$^3$-*p*-(2-(2-méthoxyéthoxy)éthoxy)-D-Phe$^4$-Lys$^5$-Val$^6$-Cys$^7$)-Thr$^8$-NH$_2$
H-2-Nal$^1$-cyclo(D-Cys$^2$-D-Tyr$^3$-Trp$^4$-D-Lys$^5$-D-Val$^6$-D-Cys$^7$)-D-Thr$^8$-NH$_2$

ou un sel pharmaceutiquement acceptable de ce composé.

**13.** Composé octapeptidique de formule générale (I) selon la revendication 12 **caractérisé en ce qu'**il s'agit d'un composé choisi parmi :

H-D-2-Nal$^1$-cyclo(Cys$^2$-Tyr$^3$-β-(3-benzothienyl)-D-Ala$^4$-Lys$^5$-Val$^6$-Cys$^7$)-Thr$^8$-NH$_2$
H-D-2-Nal$^1$-cyclo(Cys$^2$-Tyr$^3$-β-(2-thienyl)-D-Ala$^4$-Lys$^5$-Val$^6$-Cys$^7$)-Thr$^8$-NH$_2$
H-D-2-Nal$^1$-cyclo(Cys$^2$-Tyr$^3$-D-His$^4$-Lys$^5$-Val$^6$-Cys$^7$)-Thr$^8$-NH$_2$
H-D-2-Nal$^1$-cyclo(Cys$^2$-Tyr$^3$-β-(1-naphthyl)-D-Ala$^4$-Lys$^5$-Val$^6$-Cys$^7$)-Thr$^8$-NH$_2$
H-D-2-Nal$^1$-cyclo(Cys$^2$-Tyr$^3$-β-(2-fluorenyl)-D-Ala$^4$-Lys$^5$-Val$^6$-Cys$^7$)-Thr$^8$-NH$_2$
H-D-2-Nal$^1$-cyclo(Cys$^2$-Tyr$^3$-*p*-Br-D-Phe$^4$-Lys$^5$-Val$^6$-Cys$^7$)-Thr$^8$-NH$_2$
H-D-2-Nal$^1$-cyclo(Cys$^2$-Tyr$^3$-*p*-F-D-Phe$^4$-Lys$^5$-Val$^6$-Cys$^7$)-Thr$^8$-NH$_2$
H-D-2-Nal$^1$-cyclo(Cys$^2$-Tyr$^3$-D-Tyr$^4$-Lys$^5$-Val$^6$-Cys$^7$)-Thr$^8$-NH$_2$
H-D-2-Nal$^1$-cyclo(Cys$^2$-Tyr$^3$-*m*-F-D-Phe$^4$-Lys$^5$-Val$^6$-Cys$^7$)-Thr$^8$-NH$_2$
H-D-2-Nal$^1$-cyclo(Cys$^2$-Tyr$^3$-*o*-F-D-Phe$^4$-Lys$^5$-Val$^6$-Cys$^7$)-Thr$^8$-NH$_2$
H-D-2-Nal$^1$-cyclo(Cys$^2$-Tyr$^3$-*3,5*-diF-D-Phe$^4$-Lys$^5$-Val$^6$-Cys$^7$)-Thr$^8$-NH$_2$
H-D-2-Nal$^1$-cyclo(Cys$^2$-Tyr$^3$-*m*-Br-D-Phe$^4$-Lys$^5$-Val$^6$-Cys$^7$)-Thr$^8$-NH$_2$

H-D-2-$Nal^1$-cyclo($Cys^2$-$Tyr^3$-*p*-Nitro-D-$Phe^4$-$Lys^5$-$Val^6$-$Cys^7$)-$Thr^8$-$NH_2$
H-D-2-$Nal^1$-cyclo($Cys^2$-$Tyr^3$-*p*-(2-(2-méthoxyéthoxy)éthoxy)-D-$Phe^4$-$Lys^5$-$Val^6$-$Cys^7$)-$Thr^8$-$NH_2$.

**14.** Composé octapeptidique de formule générale (I) selon la revendication 12 **caractérisé en ce qu'**il s'agit d'un composé choisi parmi :

H-D-2-$Nal^1$-cyclo($Cys^2$-$Tyr^3$-D-$Ala^4$-$Lys^5$-$Val^6$-$Cys^7$)-$Thr^8$-$NH_2$
H-D-2-$Nal^1$-cyclo($Cys^2$-$Tyr^3$-Ph-D-$Gly^4$-$Lys^5$-$Val^6$-$Cys^7$)-$Thr^8$-$NH_2$.

**15.** Composé octapeptidique de formule générale (I) selon la revendication 12 **caractérisé en ce qu'**il s'agit d'un composé choisi parmi :

H-D-2-$Nal^1$-cyclo($Cys^2$-$Tyr^3$-D-$Val^4$-$Lys^5$-$Val^6$-$Cys^7$)-$Thr^8$-$NH_2$
H-D-2-$Nal^1$-cyclo($Cys^2$-$Tyr^3$-β-(2-naphthyl)-D-$Ala^4$-$Lys^5$-$Val^6$-$Cys^7$)-$Thr^8$-$NH_2$
H-D-2-$Nal^1$-cyclo($Cys^2$-$Tyr^3$-β-(9-anthryl)-D-$Ala^4$-$Lys^5$-$Val^6$-$Cys^7$)-$Thr^8$-$NH_2$
H-D-2-$Nal^1$-cyclo($Cys^2$-$Tyr^3$-homo-D-$Phe^4$-$Lys^5$-$Val^6$-$Cys^7$)-$Thr^8$-$NH_2$
H-D-2-$Nal^1$-cyclo($Cys^2$-$Tyr^3$-*p*-Ph-D-$Phe^4$-$Lys^5$-$Val^6$-$Cys^7$)-$Thr^8$-$NH_2$.

**16.** Composé octapeptidique de formule générale (I) selon la revendication 12 **caractérisé en ce qu'**il s'agit d'un composé choisi parmi :

H-D-2-$Nal^1$-cyclo($Cys^2$-$Tyr^3$-*p*-(2-(2-méthoxyéthoxy)éthoxy)-D-$Phe^4$-$Lys^5$-$Val^6$-$Cys^7$)-$Thr^8$-$NH_2$
H-2-$Nal^1$-cyclo(D-$Cys^2$-D-$Tyr^3$-$Trp^4$-D-$Lys^5$-D-$Val^6$-D-$Cys^7$)-D-$Thr^8$-$NH_2$.

**17.** Médicament comprenant un composé de formule générale (I) selon l'une des revendications 1 à 16.

**18.** Composition thérapeutique comprenant un composé de formule générale (I) selon l'une des revendications 1 à 16.

**19.** Composition thérapeutique selon la revendication 18 comprenant, à titre de principe actif, un composé de formule générale (I) selon l'une des revendications 1 à 16.

**20.** Utilisation d'un composé octapeptidique de formule générale (I) selon l'une des revendications 1 à 16 pour fabriquer un médicament.

Europäisches Patentamt
European Patent Office
Office européen des brevets

## RAPPORT DE RECHERCHE EUROPEENNE

Numéro de la demande
EP 08 29 0917

### DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | C VALERY ET AL.: "Molecular origin of the self-assembly of lanreotide into nanotubes: a mutational approach" BIOPHYSICAL JOURNAL, vol. 94, no. 5, 2008, pages 1782-1795, XP002523806 NEW YORK, US * le document en entier * ----- | 1-20 | INV. C07K14/665 A61K38/04 |
| A | Q PANDIT ET AL.: "Self-assembly of the octapeptide lanretide and lanreotide-based derivatives: the role of the aromatic residues" JOURNAL OF PEPTIDE SCIENCE., vol. 14, no. 1, 2008, pages 66-75, XP002523807 JOHN WILEY AND SONS LTD. * le document en entier * ----- | 1-20 | |
| | | | DOMAINES TECHNIQUES RECHERCHES (IPC) |
| | | | C07K A61K |

Le présent rapport a été établi pour toutes les revendications

1

| Lieu de la recherche | Date d'achèvement de la recherche | Examinateur |
|---|---|---|
| La Haye | 16 avril 2009 | Masturzo, Pietro |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.......................................................................
& : membre de la même famille, document correspondant

EPO FORM 1503 03.82 (P04C02)

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description

- **Brazeau P. et al.** *Science,* 1973, vol. 179, 77-79
- **Reisine T. et al.** *Neuroscience,* 1995, vol. 67, 777-790
- **Reisine et al.** *Endocrinology,* 1995, vol. 16, 427-442
- **Yamada et al.** *Proc. Natl. Acad. Sci. U.S.A,* 1992, vol. 89, 251-255
- **Raynor, K. et al.** *Mol. Pharmacol.,* 1993, vol. 44, 385-392
- **Moreau J.P. et al.** *Life Sciences,* 1987, vol. 40, 419
- **Harris A.G. et al.** *The European Journal of Medicine,* 1993, vol. 2, 97-105
- **Cendros JM ; Peraire C ; Trocôniz IF ; Obach R.** Pharmacokinetics and population pharmacodynamic analysis of lanreotide Autogel. *Metabolism.,* Octobre 2005, vol. 54 (10), 1276-81
- Salt selection for basic drugs. *Int. J. Pharm.,* 1986, vol. 33, 201-217
- **Valéry et al.** *Biophysical journal,* 2004, vol. 86 (4), 2484-2501
- *Proc. Natl. Acad. Sci.,* 2003, vol. 100 (18), 10258-10262
- **Cross MM.** *J Colloid Sci,* 1965, vol. 20, 417-37
- **Casson, N.** Rheology of Disperse Systems. Pergamon Press, 1959, 82